# EUROPÄISCHE PATENTSCHRIFT

(11) **EP 0 657 431 B1**
(45) Veröffentlichungstag und Bekanntmachung des Hinweises auf die Patenterteilung: **19.02.2003**
(21) Anmeldenummer: 94118672.8
(22) Anmeldetag: 28.11.1994
(51) Int. Cl.: C07D 211/90, C07D 401/12, A61K 31/44

(54) **4-Phenyl-3-substituierte 1,4-Dihydropyridinester mit cerebraler Aktivität**
4-Phenyl-3-substituted 1,4-dihydropyridine esters with cerebral activity
Esters de 4-phényle-1,4-dihydropyridine 3-substitués avec une activité cérébrale

(30) Priorität: 10.12.1993 DE 4342196
(43) Veröffentlichungstag der Anmeldung: 14.06.1995
(73) Patentinhaber: BAYER AG, 51368 Leverkusen (DE)
(72) Erfinder: Meier, Heinrich Dr., Higashi-Nada-ku, Kobe 658 (JP); Hartwig, Wolfgang Dr., Stamford, Connecticut 06903 (JP); Junge, Bodo Dr., D-42399 Wuppertal (JP); Schohe-Loop, Rudolf Dr., D-42327 Wuppertal (JP); Gao, Zhan Dr., Beijing 100016, P.R. (CN); Schmidt, Bernard Dr., D-51789 Lindlar (DE); de Jonge, Maarten Dr., D-51491 Overath (DE); Schuurman, Teunis Dr., D-53797 Lohmar (DE)

(56) Entgegenhaltungen:
- EP-A- 0 007 293
- EP-A- 0 012 180
- EP-A- 0 088 940
- EP-A- 0 525 568
- EP-A- 0 534 520
- EP-A- 0 595 164
- WO-A-88/09331
- DE-A- 2 218 644
- DE-A- 2 508 181
- US-A- 3 932 646
- JOURNAL OF CARDIOVASCULAR PHARMACOLOGY; SUPPLEMENT 1, Bd.10, 1987, NEW YORK Seiten S60 - S65 PEDER BERNTSSON ET AL. 'Felodipine Analogs: Structure - Activity Relationships'

## Beschreibung

Die Erfindung betrifft 4-Phenyl-3-substituierte 1,4-Dihydropyridinester, Verfahren zu ihrer Herstellung und ihre Verwendung in Arzneimitteln, insbesondere als cerebral wirksame Mittel.

Es ist bekannt, daß einige Dihydropyridine wie z.B. Nimodipin eine cerebrale Wirksamkeit besitzen [vgl. DOS 28 15 578]. Weiterhin sind auch Dihydropyridine bekannt, die in 4-Position einen Halogenphenylring tragen (vgl. DOS 1 963 188, DOS 2 117 572, DOS 2 117 573 und EP 007 293).

Substituierte 1,4-Dihydropyridine sind als Synthesezwischensufen bekannt (EP_A-0 088 940).

Die EP-A-0 534 520 beschreibt Verfahren zur Synthese von substituierten 1,4-Dihydropyrdinen.

Die antihypertensive und vasospasmolytische Wirkung von substituierten 1,4-Dihydropyridinen ist ebenfalls bekannt (US-A-3 932 646, DE-A-25 08 181, DE-A-22 18 644, WO-A-88/09331, J. Cardiovasc. Pharmacol. 1987, 10,S60-S65, EP-A―0 012 180).

Die EP-A-0 525 568 offenbart außerdem N-alkylierte 1,4-Dihydropyridine mit cerebraler Wirksamkeit.

Die nachveröffentlichte EP-A-0 595 164 beschreibt substituierte 4-Cyanophenyl-1,4-dihydropyridine als Zwischenstufen für die Synthese von cerebralselektiven, substituierten 4-Heteroarylphenyl-1,4-dihydropyridinen.

Die vorliegende Erfindung betrifft ausgewählte 4-Phenyl-3-substituierte 1,4-Dihydropyridinester der allgemeinen Formel (I) 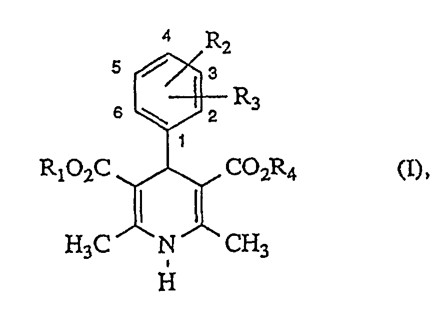 in welcher
- R¹: für geradkettiges oder verzweigtes Alkyl mit bis zu 8 Kohlenstoffatomen steht, das gegebenenfalls durch Cycloalkyl mit 3 bis 6 Kohlenstoffatomen oder durch Hydroxy oder durch geradkettiges oder verzweigtes Alkoxy mit bis zu 6 Kohlenstoffatomen substituiert ist, oder für Cycloalkyl mit 3 bis 8 Kohlenstoffatomen steht,
- R² und R³: gleich oder verschieden sind und für Halogen oder Cyano stehen, oder
- R² oder R³: für Wasserstoff steht,
- R⁴: für geradkettiges oder verzweigtes Alkyl mit bis zu 8 Kohlenstoffatomen steht, das durch Cycloalkyl mit 3 bis 6 Kohlenstoffatomen,
oder durch einen Rest der Formel

-OR⁵ ,

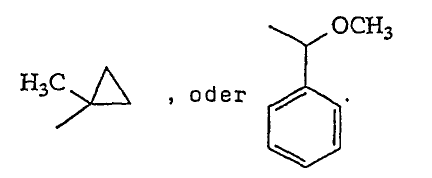

substituiert ist,
worin
R⁵ Cycloalkyl mit 3 bis 6 Kohlenstoffatomen, geradkettiges oder verzweigtes Fluoralkyl mit bis zu 5 Fluoratomen oder geradkettiges oder verzweigtes Alkyl mit bis zu 8 Kohlenstoffatomen bedeutet, das seinerseits durch Methoxy, Ethoxy oder Cycloalkyl mit 3 bis 7 Kohlenstoffatomen substituiert ist
und deren physiologisch unbedenkliche Salze,
mit der Maßgabe, daß R⁴ nicht -(CH₂CH₂O)₂CH₃ bedeutet, wenn
R¹ für -C₂H₅, -CH(CH₃)₂, -C(CH₃)₃, -CH(CH₃)C₂H5, -CH₂CH₂OCH(CH₃)₂, -CH(CH₃)CH₂OCH₃ oder C(CH₃)₂CH₂OCH₃ steht, und
R² und R³ für Chlor stehen.
Physiologisch unbedenkliche Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder. Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- R¹: für geradkettiges oder verzweigtes Alkyl mit bis zu 6 Kohlenstoffatomen steht, das gegebenenfalls durch Cyclopropyl, Cyclopentyl, Cyclohexyl, Hydroxy oder durch geradkettiges oder verzweigtes Alkoxy mit bis zu 5 Kohlenstoffatomen substituiert ist, oder
für Cyclopentyl, Cyclohexyl oder Cycloheptyl steht,
- R² und R³: gleich oder verschieden sind und für Fluor, Chlor, Brom oder Cyano stehen, oder
- R² oder R³: für Wasserstoff steht,
- R⁴: für geradkettiges oder verzweigtes Alkyl mit bis zu 6 Kohlenstoffatomen steht, das durch Cyclopropyl, Cyclopentyl, Cyclohexyl oder durch einen Rest der Formel

-OR⁵ ,

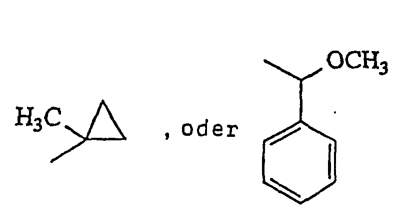

substituiert ist,
worin
R⁵ Cyclopropyl, Cyclopentyl, Cyclohexyl, geradkettiges oder verzweigtes Fluoralkyl mit bis zu 4 Fluoratomen oder geradkettiges oder verzweigtes Alkyl mit bis zu 6 Kohlenstoffatomen bedeutet, das seinerseits durch Methoxy, Ethoxy oder Cyclopropyl substituiert ist
und deren physiologisch unbedenkliche Salze,
mit der Maßgabe, daß R⁴ nicht -(CH₂CH₂O)₂CH₃ bedeutet, wenn
R¹ für -C₂H₅, -CH(CH₃)_{2,} -C(CH₃)₃, -CH(CH₃)C₂H5, -CH₂CH₂OCH(CH₃)₂, -CH(CH₃)CH₂OCH₃ oder C(CH₃)₂CH₂OCH₃ steht, und
R² und R³ für Chlor stehen.

Besonders bevorzugt sind Verbindungen der allgemeinen Formel (I),
in welcher
- R¹: für geradkettiges oder verzweigtes Alkyl mit bis zu 5 Kohlenstoffatomen steht, das gegebenenfalls durch Cyclopentyl, Cyclohexyl oder durch Hydroxy oder geradkettiges oder verzweigtes Alkoxy mit bis zu 4 Kohlenstoffatomen substituiert ist, oder
für Cyclopentyl, Cyclohexyl oder Cycloheptyl steht,
- R² und R³: gleich oder verschieden sind und für Fluor, Chlor, Brom oder Cyano stehen, oder
entweder R² oder R³ für Wasserstoff steht,
- R⁴ für: geradkettiges oder verzweigtes Alkyl mit bis zu 5 Kohlenstoffatomen steht, das durch Cyclopropyl, Cyclopentyl, Cyclohexyl, oder durch einen Rest der Formel

-OR⁵ ,

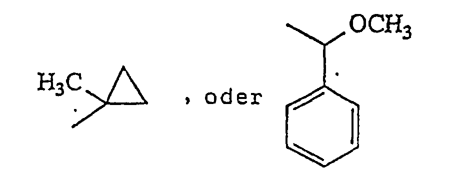

substituiert ist,
worin
R⁵ Cyclopropyl, Cyclopentyl, Cyclohexyl, geradkettiges oder verzweigtes Fluoralkyl mit bis zu 3 Fluoratomen oder geradkettiges oder verzweigtes Alkyl mit bis zu 5 Kohlenstoffatomen bedeutet, das seinerseits durch Methoxy oder Cyclopropyl substituiert ist.
und deren physiologisch unbedenkliche Salze
mit der Maßgabe, daß R⁴ nicht -(CH₂CH₂O)₂CH₃ bedeutet, wenn
R¹ für -C₂H₅, -CH(CH₃)₂, -C(CH₃)₃, -CH(CH₃)C₂H5, -CH₂CH₂OCH(CH₃)₂, -CH(CH₃)CH₂OCH₃ oder C(CH₃)₂CH₂OCH₃ steht, und
R² und R³ für Chlor stehen.

Verbindungen gemäß Formel (I) sind solche aus der Gruppe
Cyclohexylmethyl-2-methoxyethyl-4-(3,4-difluor-phenyl)-1,4-dihydro-2,6-dimethylpyridin-3,5-dicarboxylat,
2-Cyclohexylethyl-2-methoxyethyl-4-(3,4-difluor-phenyl)-1,4-dihydro-2,6-dimethylpyridin-3,5-dicarboxylat,
3-Cyclohexylpropyl-2-methoxyethyl-4-(3,4-difluor-phenyl)-1,4-dihydro-2,6-dimethylpyridin-3,5-dicarboxylat,
Isopropyl-2-(2-methoxyethoxy)ethyl-4-(2-chlor-6-fluor-phenyl)-1,4-dihydro-2,6-dimethylpryridin-3,5-dicarboxylat,
Cyclopentyl-2-(2-methoxyethoxy)ethyl-4-(2-chlor-6-fluor-phenyl)-1,4-dihydro-2,6-dimethylpyridin-3,5-dicarboxylat,
2-Cyclohexyloxyethyl-isopropyl-4-(3-cyano-phenyl)-1,4-dihydro-2,6-dimethylpyridin-3,5-dicarboxylat,
2-Cyclohexyloxyethyl-cyclopentyl-4-(3-cyano-phenyl)-1,4-dihydro-2,6-dimethylpyridin-3,5-dicarboxylat,
Cyclohexylmethyl-2-methoxyethyl-4-(3-cyano-phenyl)-1,4-dihydro-2,6-dimethylpyridin-3,5-dicarboxylat,
Cyclopropylmethyl-2-methoxyethyl-4-(3-chlor-2-cyano-phenyl)-1,4-dihydro-2,6-dimethylpyridin-3,5-dicarboxylat,
2-Methoxyethyl-(1-methyl-cyclopropyl)methyl-4-(3-chlor-2-cyano-phenyl)-1,4-dihydro-2,6-dimethylpyridin-3,5-dicarboxylat,
Isopropyl-(S)-methoxy-2-phenylethyl-4-(2,3-difluor-phenyl)-1,4-dihydro-2,6-dimethylpyridin-3,5-dicarboxylat,
Cyclopropyl-(S)-2-methoxy-2-phenylethyl-4-(2,3-difluor-phenyl)-1,4-dihydro-2,6-dimethylpyridin-3,5-dicarboxylat,
Isopropyl-(R)-2-methoxy-2-phenylethyl-4-(2,3-difluor-phenyl)-1,4-dihydro-2,6-dimethylpyridin-3,5-dicarboxylat,
Cyclopentylmethyl-2-methoxyethyl-4-(2,3-difluor-phenyl)-1,4-dihydro-2,6-dimethylpyridin-3,5-dicarboxylat,
Cyclohexylmethyl-2-methoxyethyl-4-(2,3-difluor-phenyl)-1,4-dihydro-2,6-dimethylpyridin-3,5-dicarboxylat,
Cyclohexylmethyl-2-methoxypropyl-4-(2,3-difluor-phenyl)-1,4-dihydro-2,6-dimethylpyridin-3,5-dicarboxylat,

Außerdem wurden Verfahren zur Herstellung der erfindungsgemäßen Verbindungen der allgemeinen Formel (I) gefunden, dadurch gekennzeichnet, daß man
[A] Aldehyde der allgemeinen Formel (II) 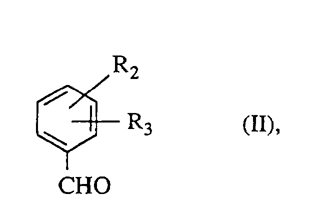 in welcher
   R² und R³ die angegebene Bedeutung haben,
   zunächst mit Acetessigestem der allgemeinen Formel (III)

   H₃C-CO-CH₂-CO₂R¹ (III),

   in welcher
   R¹ die angegebene Bedeutung hat,
   gegebenenfalls unter Isolierung der entsprechenden Ylidenverbindungen der allgemeinen Formel (IV) 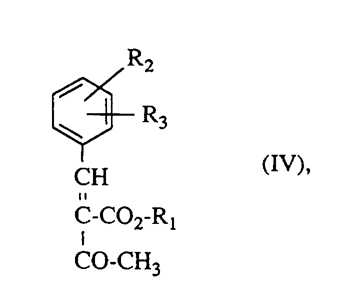 in welcher
   R¹, R² und R³ die angegebene Bedeutung haben,
   umsetzt und anschließend entweder mit Verbindungen der allgemeinen Formel (V)

   CH₃-CO-CH₂-CO₂R⁴ (V),

   in welche
   R⁴ die angegebene Bedeutung hat,
   in inerten Lösemitteln, in Anwesenheit von Ammoniak oder Ammoniumsalzen,
   oder direkt mit Enaminoderivaten der allgemeinen Formel (VI) 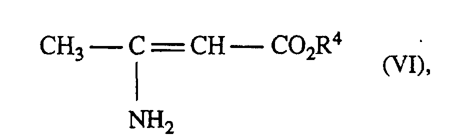 in welcher
   - R⁴: die angegebene Bedeutung hat,
   umsetzt,
   oder
[B] die Aldehyde der allgemeinen Formel (II) zunächst mit den Verbindungen der allgemeinen Formel (V), gegebenenfalls unter Isolierung der Ylidenverbindungen der allgemeinen Formel (VII) 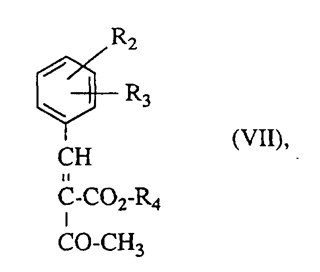 in welcher
   R², R³ und R⁴ die angegebene Bedeutung haben,
   umsetzt und in einem nächsten Schritt mit den Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII) 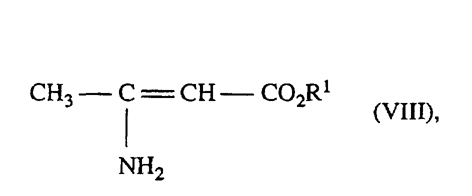 in welcher
   R¹ die angegebene Bedeutung hat,
   umsetzt,
   oder
[C] Verbindungen der allgemeinen Formel (IX) 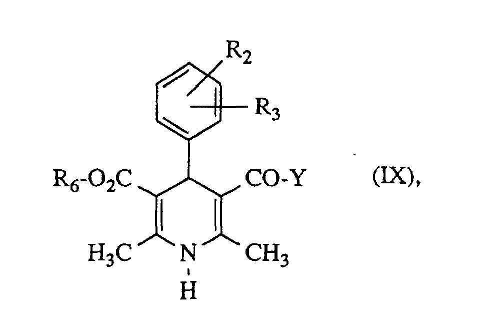 in welcher
   - R² und R³: die angegebene Bedeutung haben,
   - R⁶: den angegebenen Bedeutungsumfang von R¹ oder R⁴ umfaßt,
   und
   - Y: zusammen mit der -CO-Gruppe eine reaktives Carbonsäurederivat bildet,
   in inerten Lösemitteln, in Anwesenheit einer Base,
   mit Verbindungen der allgemeinen Formel (X)

   R⁷-OH (X),

   in welcher
   - R⁷: die angegebene Bedeutung von R⁶ hat,
umsetzt,
und im Fall der reinen Ester-Enantiomere, die enantiomerenreinen Carbonsäuren, gegebenenfalls zunächst über die Stufe eines reaktiven Säurederivates, mit den entsprechenden Alkoholen umsetzt.

Das erfindungsgemäße Verfahren kann durch folgendes Formelschema beispielhaft erläutert werden:

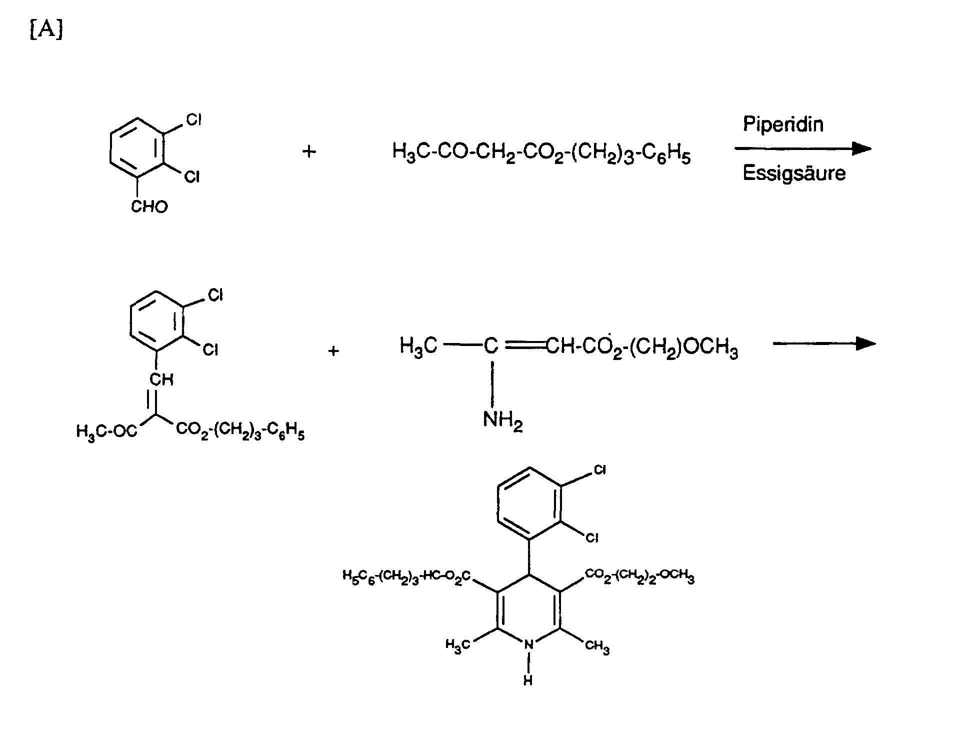

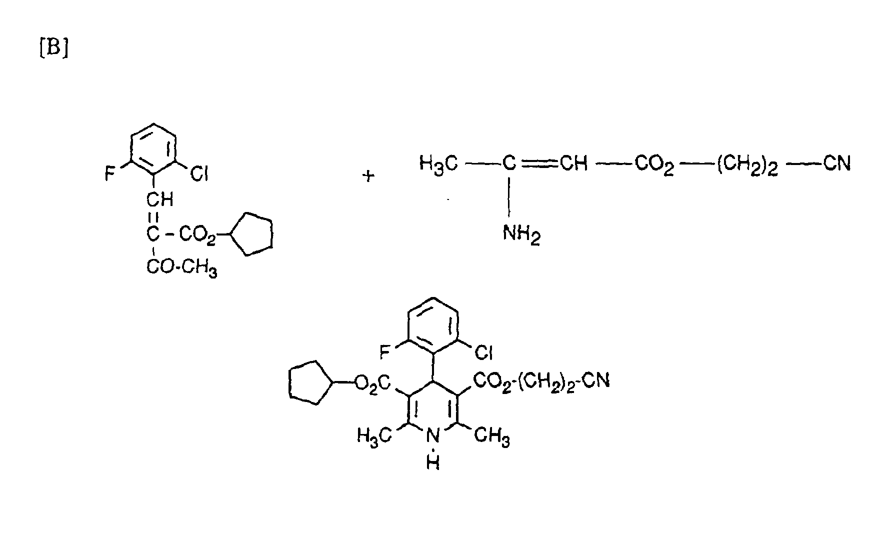

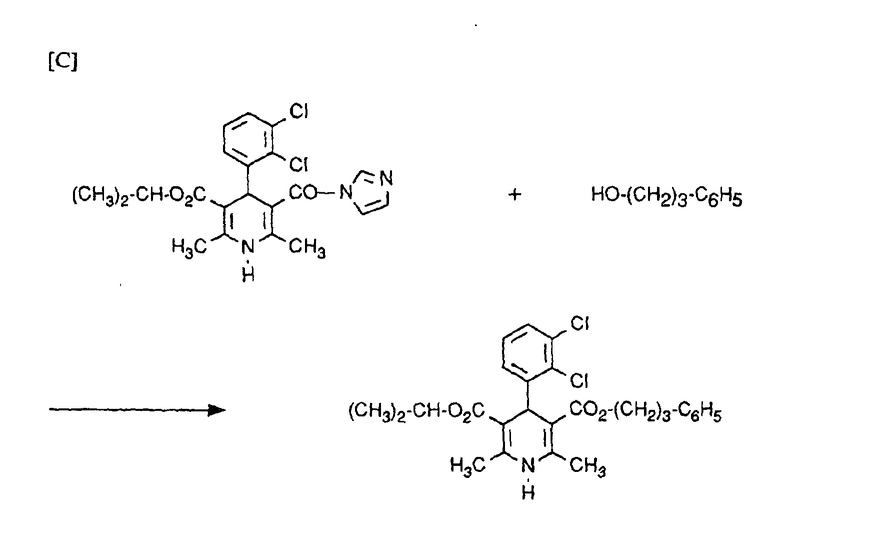

Als Lösemittel für die Verfahren [A] und [B] eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind Isopropanol, Tetrahydrofuran, Methanol, Dioxan und Dimethylformamid.

Als Lösemittel für das Verfahren [C] eignen sich die oben aufgeführten Lösemittel mit Ausnahme der Alkohole und Essigsäure.

Als Basen für die Aktivierung der Carbonsäuren eignen sich im allgemeinen Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind in Abhängigkeit der jeweiligen Reaktionsschritte Piperidin, Dimethylaminopyridin, Pyridin, Natriumhydrid und Kalium-tert.butylat.

Als Hilfsstoffe werden bevorzugt Kondensationsmittel eingesetzt die auch Basen sein können. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphonat. Bevorzugt sind N,N'-Dicyclohexylcarbodiimid und Carbonyldiimidazol.

Als Basen eignen sich im allgemeinen Alkalicarbonate wie beispielsweise Natriumoder Kaliumcarbonat, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo[5.4.0]undec-7-en (DBU) oder 1,5-Diazabicyclo-[4.3.0]non-5-en (DBN). Bevorzugt ist Dimethylaminopyridin.

Die Base wird im allgemeinen in einer Menge von 0,01 mol bis 1 mol, bevorzugt von 0,05 mol bis 0,1 mol, jeweils bezogen auf 1 mol der Verbindungen der allgemeinen Formeln (II) oder (IX), eingesetzt.

Die Hilfsstoffe werden im allgemeinen in einer Menge von 1 mol bis 3 mol, bevorzugt von 1 mol bis 1,5 mol, jeweils bezogen auf 1 mol der Verbindungen der allgemeinen Formeln (II) und (IX), eingesetzt.

Die Reaktionstemperatur für die Verfahren [A] und [B] können in einem größeren Bereich variiert werden. Im allgemeinen arbeitet man in einem Bereich von -20°C bis 200°C, bevorzugt von 0°C bis 100°C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B. außerdem, indem man Diastereomerengemische der Verbindungen der allgemeinen Formel (I), in welcher R¹ oder R⁴ sich aus einem enantiomerenreinen chiralen Alkohol ableitet, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen.
Die Veresterung der enantiomerenreinen Dihydropyridine erfolgt bevorzugt in Ethem wie Diethylether oder Tetrahydrofuran, Dimethylformamid, Methylenchlorid, Chloroform, Acetonitril oder Toluol.

Die Aldehyde der allgemeinen Formel (II) sind an sich bekannt oder können nach üblichen Methoden hergestellt werden.

Ebenso sind die Acetessigester der allgemeinen Formeln (III) und (V) und die Enaminoderivate der allgemeinen Formeln (VI) und (VIII) bekannt.

Die reaktiven Säurederivate der allgemeinen Formel (IX) sind teilweise bekannt oder neu und können dann nach üblichen Methoden hergestellt werden.

Die Verbindungen der allgemeinen Formel (X) sind an sich bekannt.

Die Verbindungen der allgemeinen Formel (IV) und (VII) sind größenteils bekannt oder können nach üblichen Methoden hergestellt werden. Die Herstellung der Verbindungen der allgemeinen Formel (I) ist nicht auf diese Verfahren beschränkt, sondern jede Modifikation dieser Verfahren ist in gleicher Weise für die Herstellung der erfindungsgemäßen Verbindungen anwendbar.

Ein weiterer Aspekt der Erfindung betrifft die Verwendung von
4-Phenyl-3-substituierte 1,4-Dihydropyridinester der allgemeinen Formel 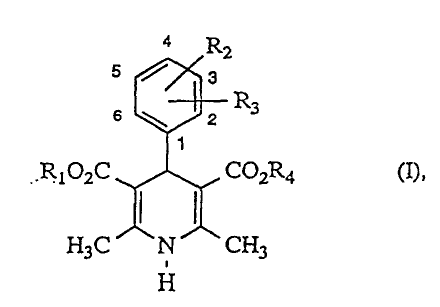 in welcher
- R¹: für geradkettiges oder verzweigtes Alkyl mit bis zu 8 Kohlenstoffatomen steht, das gegebenenfalls durch Cycloalkyl mit 3 bis 6 Kohlenstoffatomen oder durch Hydroxy oder durch geradkettiges oder verzweigtes Alkoxy mit bis zu 6 Kohlenstoffatomen substituiert ist, oder
für Cycloalkyl mit 3 bis 8 Kohlenstoffatomen steht,
- R² und R³: gleich oder verschieden sind und für Halogen oder Cyano stehen, oder
- R² oder R³: für Wasserstoff steht,
- R⁴: für geradkettiges oder verzweigtes Alkyl mit bis zu 8 Kohlenstoffatomen steht, das durch Cycloalkyl mit 3 bis 6 Kohlenstoffatomen,
oder durch einen Rest der Formel

-OR⁵ ,

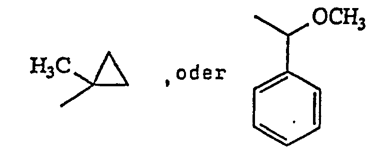

substituiert ist, worin
R⁵ Cycloalkyl mit 3 bis 6 Kohlenstoffatomen, geradkettiges oder verzweigtes Fluoralkyl mit bis zu 5 Fluoratomen oder geradkettiges oder verzweigtes Alkyl mit bis zu 8 Kohlenstoffatomen bedeutet, das seinerseits durch Methoxy, Ethoxy oder Cycloalkyl mit 3 bis 7 Kohlenstoffatomen substituiert ist,
zur Herstellung von Arzneimitteln zur Behandlung von zentral degenerativen Erkrankungen von Hirnleistungs- und Gedächtnisstörungen, cerebralen Durchblutungsstörungen und Depressionen.

Die erfindungsgemäßen Verbindungen zeigen ein nicht vorhersehbares, wertvolles pharmakologisches Wirkspektrum.

Die erfindungsgemäßen Verbindungen sind Calciumkanalliganden mit Selektivität für L-Typ-Calciumkanäle des zentralen Nervensystems. Diese Selektivität läßt sich z.B. durch Vergleich der Bindungsaffinitäten zu DHP-Bindungstellen an Rattenhirn und Rattenherz zeigen.

Die Verbindungen beeinflussen positiv Lern- und Gedächtnisleistungen, wie ihre leistungsverbessernde Wirkung auf Ratten in typischen Lem- und Gedächtnismodellen wie Wasser-Labyrinth, Morris-Labyrinth, Passives Vermeiden, Reminiszenztests in automatisierten Skinner-Boxen demonstriert.Sie besitzen ein antidepressives Potential, wie ihre Wirksamkeit im Rattenschwimmtest nach Porsolt belegt.

### Bindungsassays:

Die Bindungsaffinitäten zu PN 200-110-Bindungsstellen im Rattenhimen bzw. Rattenherzen wurden nach Rarnpe D.R., Mutledge A., Janis R.A., Triggle D.J.: Can. Journ. Physiol. Pharmacol. 65, (1987) 1452 bestimmt.

### Wasserlabyrinth:

Alte Wistar-Ratten (24 Monate) werden an die Startposition in einem mit kaltem (14-15°) Wasser gefüllten, durch senkrechte Barrieren unterteilten Plastiktank (120x50x40 cm) gesetzt Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichet. Dabei wird ein Fehler definiert als Schwimmen in eine Sackgasse oder Schwimmen über die Grenzlinie imaginärer Quadrate, in die der Tank unterteilt ist, in die Richtung fort vom Ausstieg.
Die Ratten bleiben bis zum Finden des Ausgangs, längstens aber 300 sec. im Labyrinth. Dann werden sie aufgenommen, getrocknet und unter einem Rotlicht gewärmt. Danach kehren sie in ihre Heimatkäfige zurück.
In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo sind Verkürzung der Zeit bis zum Erreichen des Ausstiegs, Verringerung der Zahl der Fehler und Vergrößerung der Zahl der Tiere,die den Ausstieg überhaupt finden.

### Rattenschwimmtest nach Porsolt

Während eines Vortests werden junge Ratten in einen Glaszylinder (40 cm hoch, 20 cm Durchmesser) gesetzt, der 17 cm hoch mit Wasser von 25°C gefüllt ist. Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral despair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet. Dabei wird eine Ratte als immobil angesehen, die aufrecht im Wasser treibend nur noch minimale Bewegungen ausführt, um den Kopf über Wasser zu halten. Placebo bzw. Testsubstanzen (0,25, 0,5, 1, 5, 10 mg/kg; n = 6 je Gruppe) werden dreimal per os verabreicht: 23, 5 und 1 h vor der Testsitzung (1, 19, 23 h nach dem Vortest). Die antidepressive Wirkung der Testsubstanzen zeigt sich in der Reduktion der Immobilitätsdauer im Vergleich zu den Placebowerten.

Aufgrund ihrer pharmakologischen Eigenschaften können sie für die Herstellung von Arzneimitteln zur Behandlung von zentral degenerativen Erkrankungen eingesetzt werden, wie sie z.B. auftreten bei Demenzen (Multiinfarktdemenz MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignen sich die Wirkstoffe zur Behandlung von Hirnleistungsstörungen im Alter, des hirnorganischen Psychosyndroms (HOPS, Organic brain syndrom, OBS) und von altersbedingten Gedächtnisstörungen (age associated memory impairment, AAMI).

Sie sind wertvoll zur Prophylaxe und zur Bekämpfung der Folgen cerebraler Durchblutungsstörungen wie cerebraler Ischämien, Schlaganfällen und von Subarachnoidalblutungen.

Sie sind geeignet zur Behandlung von Depressionen und der Manie. Weitere Anwendungsgebiete sind die Behandlung von Migräne, von Neuropathien, die z.B. durch Traumata, metabolische Erkrankungen wie Diabetes mellitus, Vergiftungen, Mikroorganismen oder Autoimmunerkrapkungen verursacht werden, von Suchterkrankungen und Entzugserscheinungen.

Die erfindungsgemäßen Verbindungen sind Ca²⁺-Antagonisten mit Selektivität für L-Typ-Ca²⁺-Kanäle des zentralen Nervensystems.
Diese Selektivität übertrifft die der bekannten cerebral wirksamen Ca²⁺-antagonistischen Dihydropyridine Nimodipin und Nicardipin. Dies zeigt sich z.B. beim Vergleich der Bindungsaffinitäten zu DHP-(PN-200 110)-Bindungsstellen in Rattenhim und Rattenherz [vgl. Rampe, D.R., Rutledge, A., Janis, R.A., Triggle, D.J., Can. Journ. Physiol. Pharmacol. 65 (1987), 1452].

| Bsp.-Nr. | Kᵢ(Hirn)[nM] | Kᵢ(Herz)[nM] | Selektivität |
|---|---|---|---|
| Nimodipin | 2,4 | 4,6 | 1,9 |
| Nicardipin | 32 | 14 | 0,44 |
| 21 | 13 | 78 | 6,0 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 0,1 mg/kg bis 20 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung oder mit Molybdatophosphorsäurelösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in einem steigenden Ausmaß die polare Laufmittelkomponente zugemischt, bis das gewünschte Produkt eluiert wird (DC-Kontrolle).

Bei allen Produkten wurde bei zuletzt ca. 0,1 Torr das Lösemittel abdestilliert.

### Ausgangsverbindungen

### Beispiel I

### 2-Acetyl-3-(2,4-difluorphenyl)-2-propensäure-2-methoxyethylester

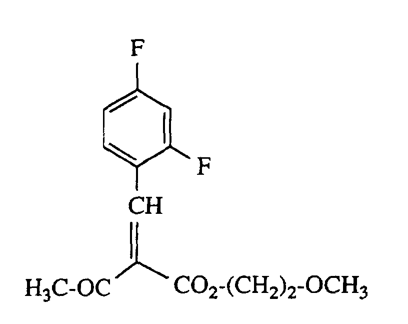

5,0 g (35 mmol) 2,4-Difluorbenzaldehyd werden mit 5,7 g (35 mmol) Acetessigsäure-2-methoxyethylester in 100 ml Isopropanol gelöst. Dazu gibt man eine frisch bereitete Lösung von 1,0 ml Piperidin und 0,5 ml Eisessig in 5 ml Isopropanol und rührt über Nacht bei 40°C. Man engt das Gemisch ein, nimmt in Toluol auf, engt erneut ein und reinigt durch Filtration über 100 ml Kieselgel (Laufmittel: Toluol /Essigester 100:1) zu 5 g der Zielverbindung als gelbes Öl, das unmittelbar weiter umgesetzt wird.

### Beispiel II

### 4-(2-Chlor-6-fluorphenyl)-1,4-dihydro-2,6-dimethylpyridin-3,5-dicarbonsäuremonocyclopentylester

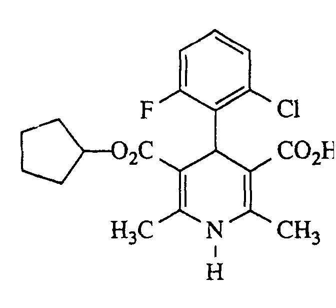

98 g (0,22 mol) 4-(2-Chlor-6-fluorphenyl)-1,4-dihydro-2,6-dimethylpyridin-3,5-dicarbonsäure-(2-cyanoethyl)-cyclopentylester werden in 400 ml 1,2-Dimethoxyethan gelöst und mit 400 ml 1 N Natronlauge über Nacht bei Zimmertemperatur gerührt Man reduziert das Lösemittelvolumen auf etwa die Hälfte, wäscht mit Dichlormethan und säuert die wäßrige Phase mit 2 N Salzsäure an (pH = 2). Zweifache Extraktion mit Dichlormethan, Waschen der organischen Phasen mit Wasser, Trocknen über Natriumsulfat, Einengen und Kristallisation aus Ether ergibt 42 g der Zielverbindung als Feststoff vom Schmelzpunkt ca. 120°C (Zers.).

### Beispiel III

### 4-(2-Chlor-6-fluorphenyl)-1,4-dihydro-5-( 1-imidazolylcarbonyl)-2,6-dimethylpyridin-3-carbonsäurecyclopentylester

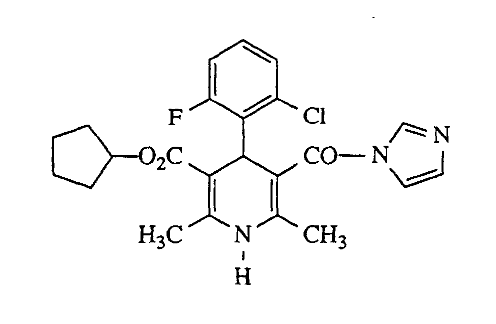

Zu 33,0 g (83 mmol) 4-(2-Chlor-6-fluorphenyl)-1,4-dihydro-2,6-dimethylpyridin-3,5-dicarbonsäuremonocyclopentylester in 350 ml Tetrahydrofuran gibt man 13,6 g (83 mmol) Carbonyldiimidazol und erhitzt 3 h zu Rückfluß. Dünnschichtchromatographische Kontrolle (Kieselgel, Toluol / Essigester 1:1) zeigt vollständigen Umsatz, woraufhin das Reaktionsgemisch eingeengt, in Essigester aufgenommen, zweimal mit Wasser gewaschen, über Natriumsulfat getrocknet und erneut eingeengt wird. Aus Ether fällt die Zielverbindung in Form weißer Kristalle vom Schmp. 150°C
Ausbeute: 29,7 g

### Herstellungsbeispiele

### Beispiel 1

### 2-Cyanoethyl-cyclopentyl-4-(2-chlor-6-fluorphenyl)-1,4-dihydro-2,6-dimethylpyridin-3,5-dicarboxylat

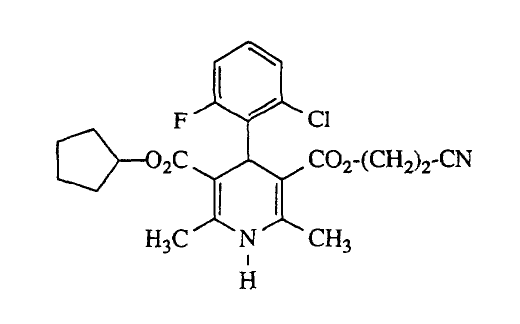

280 g (0,63 mol) 2-Acetyl-3-(2-chlor-6-fluorphenyl)-2-propensäure-cyclopentylester werden mit 97,2 g (0,63 mol) 3-Amino-2-butensäure-2-cyanoethylester in 400 ml Isopropanol gelöst und für ca. 8 h zum Rückfluß erhitzt, bis dünnschichtchromatographische Kontrolle (Kieselgel, Toluol / Essigester 5:1) vollständigen Umsatz zeigt. Das Reaktionsgemisch wird eingeengt, noch zweimal mit Toluol aufgenommen und wieder eingeengt. Zweifache Filtration über je 2 l Kieselgel mit einem Toluol / Essigester-Gradienten ergibt 160 g der Zielverbindung als Öl. Ein Teil wird durch Chromatographie an Kieselgel und Kristallisation aus Ether weiter gereinigt zu einem kristallinen Feststoff vom Schmp. 135-136°C.

### Beispiel 2

### Cyclopentyl-(2-fluorethyl)-4-(2-chlor-6-fluorphenyl)-1,4-dihydro-2,6-dimethylpyridin-3,5-dicarboxylat

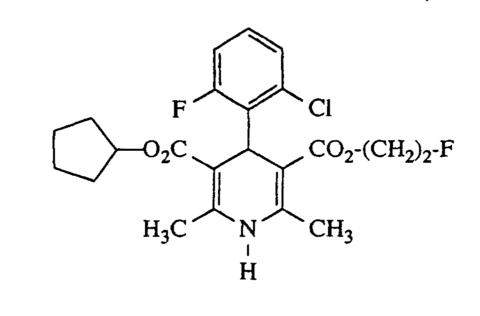

2,0 g der Verbindung aus Beispiel III werden in 20 ml Fluorethanol 12 h bei 100°C gerührt, dann der überschüssige Alkohol abdestilliert. Das verbleibende Rohprodukt wird durch Filtration über Kieselgel in Toluol und durch Chromatographie an Kieselgel in Dichlormethan / Essigester 20:1 gereinigt Man erhält 0,9 g der Zielverbindung in Form eines Schaums.
Smp.: 55°C
R_{f} (SiO₂, Toluol / Essigester 5/1) = 0,30

In Analogie zu den Vorschriften der Beispiele 1 und 2 werden die in den Tabellen 1, 2 und 3 aufgeführten Verbindungen hergestellt:

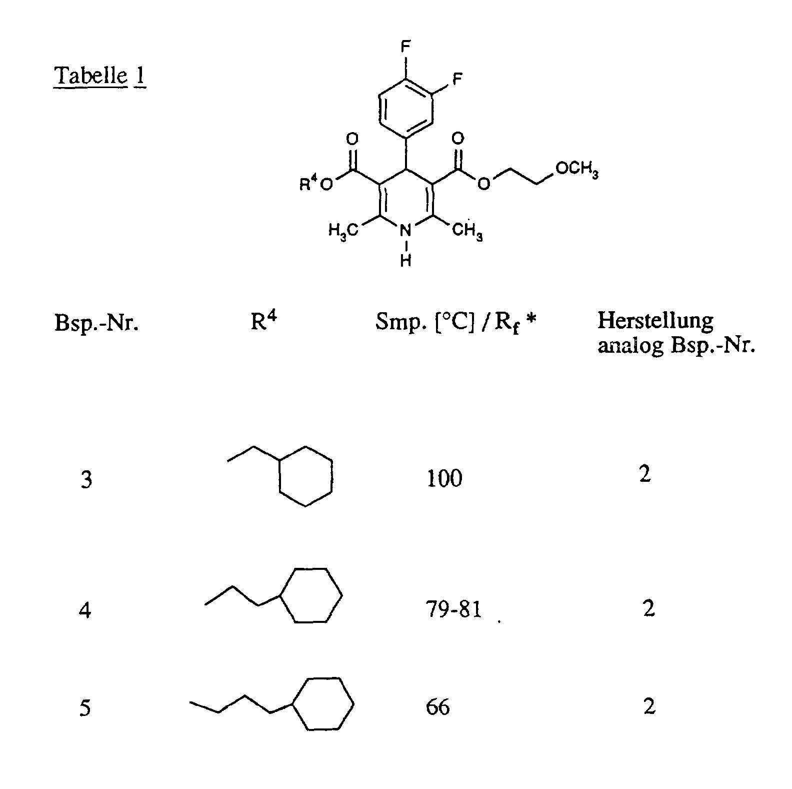

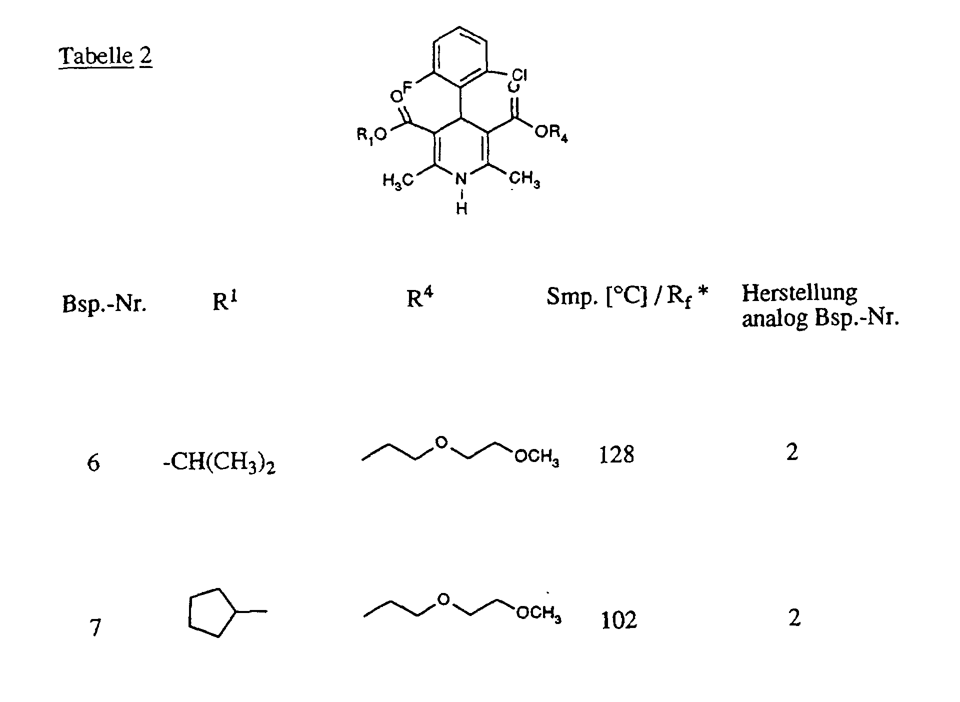

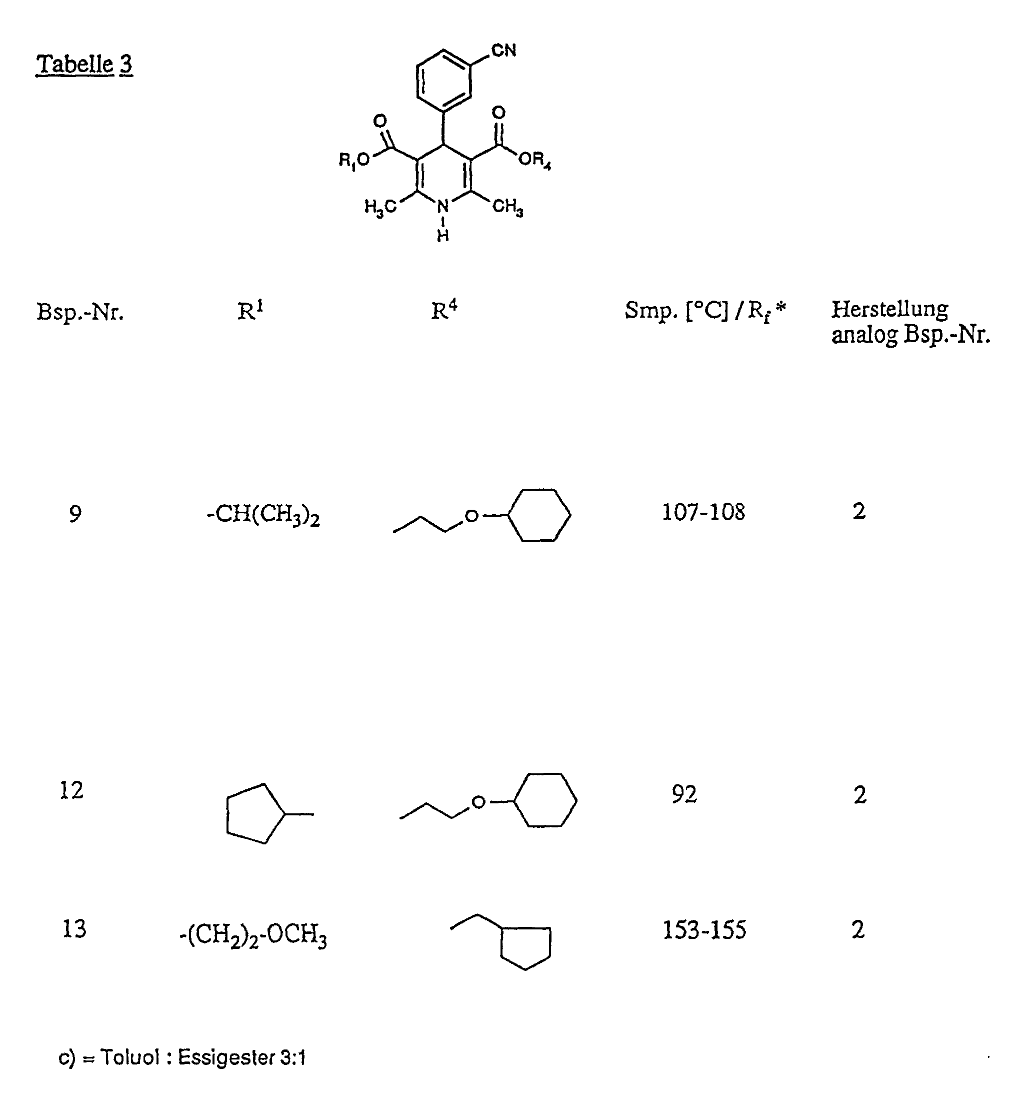

### Beispiel 14

### 5-(3-Phenylpropoxycarbonyl)-2,6-dimethyl-4-(2,3-dichlorphenyl)-1,4-dihydropyridin-3-carbonsäureisopropylester

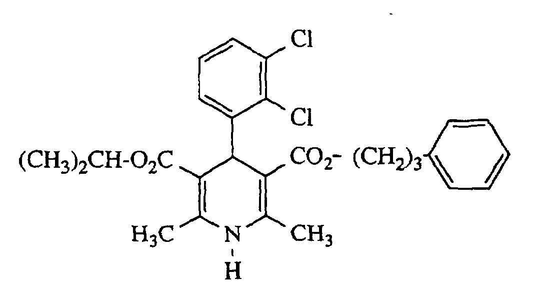

4,34 g (10 mmol) 4-(2,3-Dichlorphenyl)-1,4-dihydro-2,6-dimethylpyridin-3-isopropoxycarbonyl-3-carbonsäure-imidazolid, 1,63 g (12 mmol) 3-Phenylpropanol und 360 mg (12 mmol) NaH (80%ige Suspension) werden in 30 ml abs. THF 1 h bei 40°C gerührt (DC-Kontrolle). Das Lösemittel wird im Vakuum abdestilliert, der Rückstand in 30 ml Essigsäureethylester aufgenommen und mit 2 N HCl-Lösung und gesättigter NaCl-Lösung gewaschen. Nach Trocknen über Na₂SO₄ wird eingedampft und der Rückstand durch Chromatographie über Kieselgel gereinigt (Eluens: Toluol / Essigester 10:1).
Ausbeute: 43,1%
R_{f} = 0,36 (Tol / EE 5:1)

In Analogie zu den Vorschriften der Beispiele 1, 2 und 14 werden die in der Tabelle 4 aufgeführten Verbindungen hergestellt:

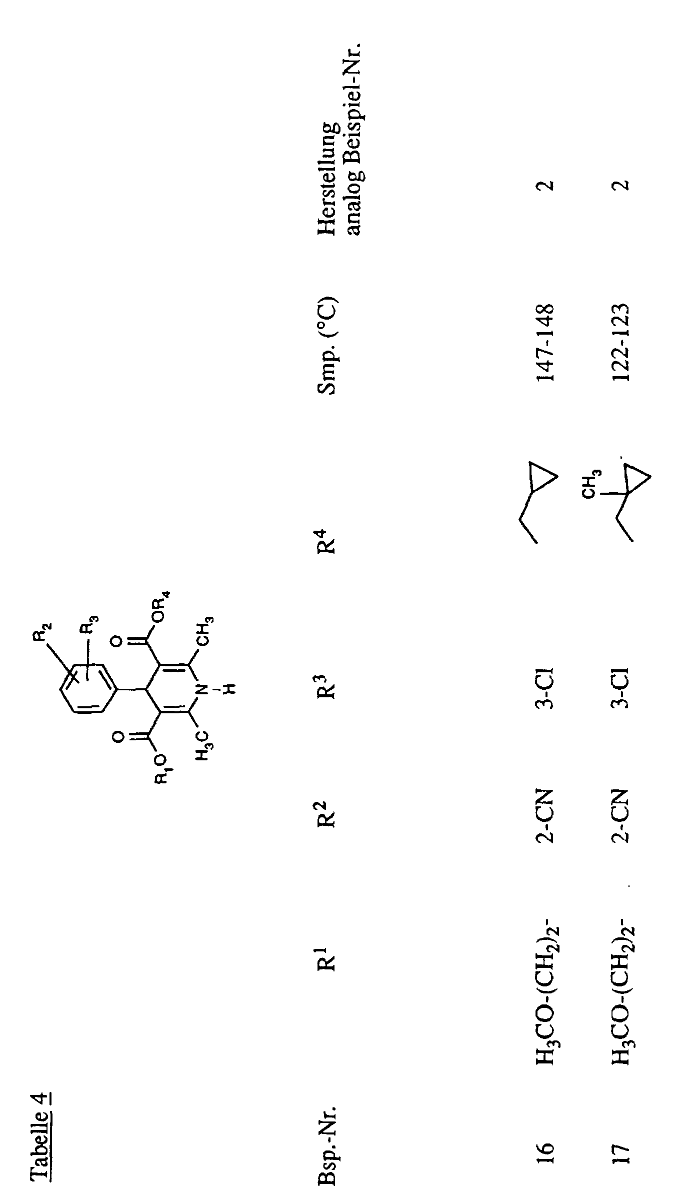

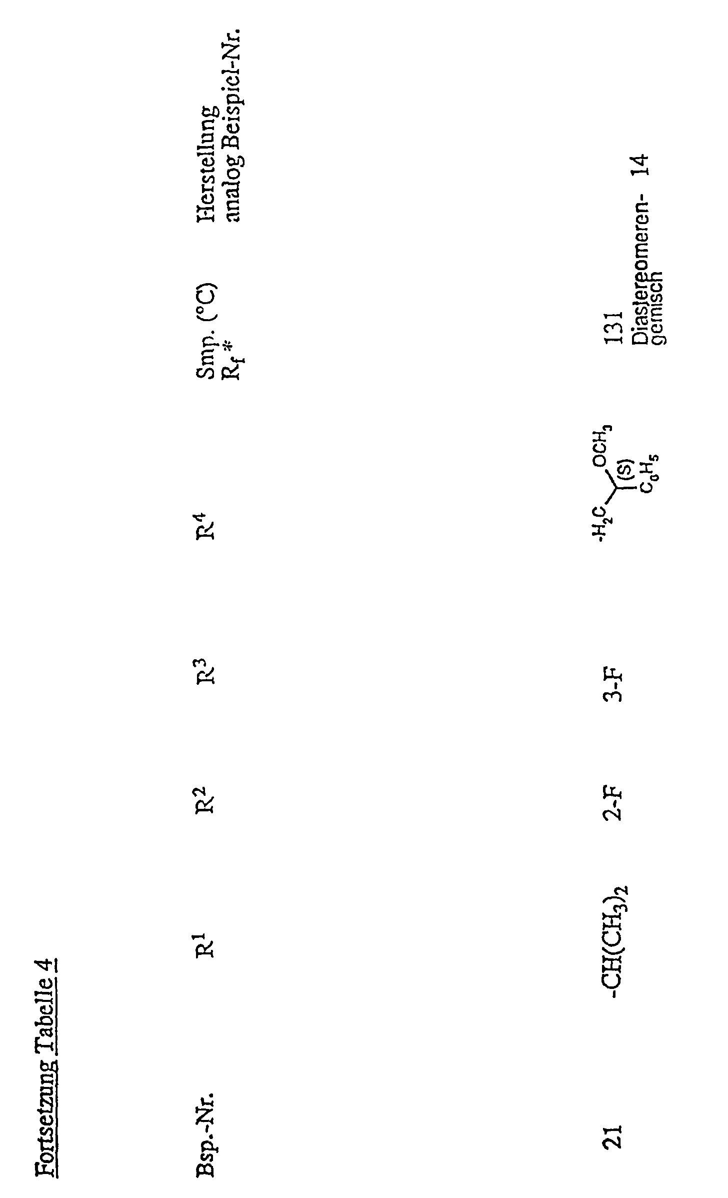

Die in Tabelle 5 aufgeführten Verbindungen werden in Analogie zur Vorschrift des Beispiels 14 aus dem entsprechenden Imidazolid hergestellt:

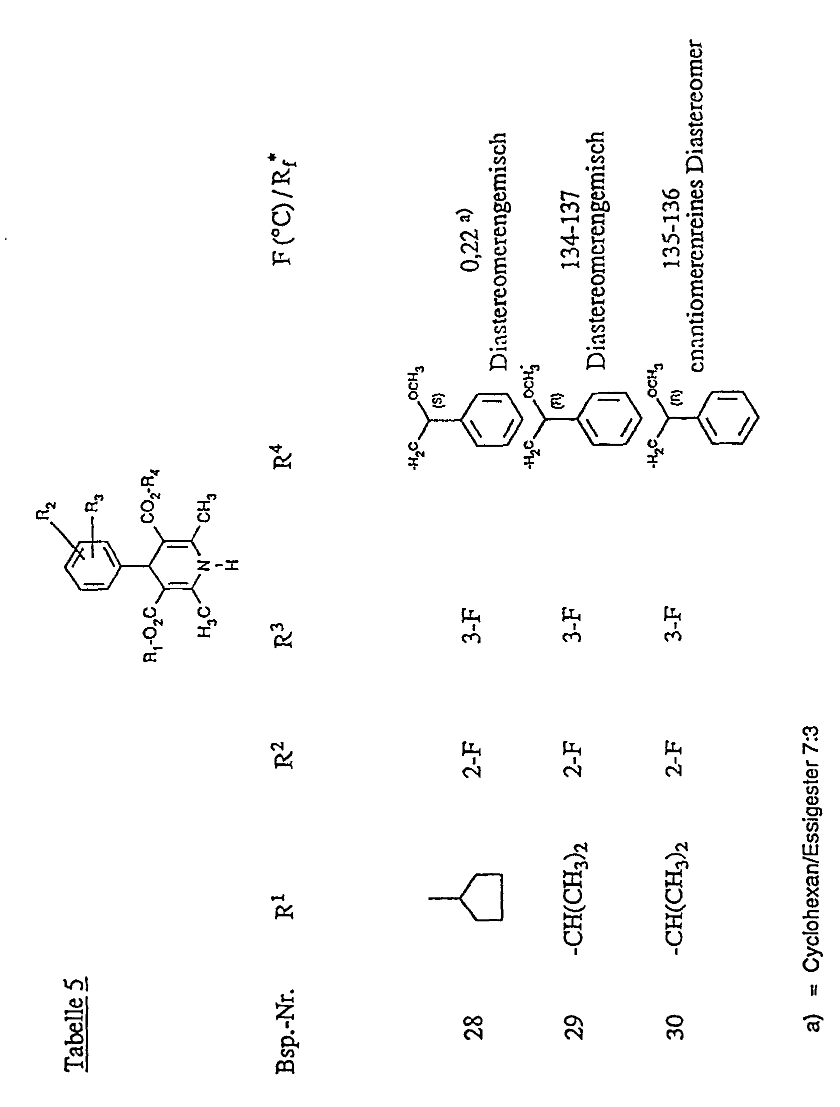

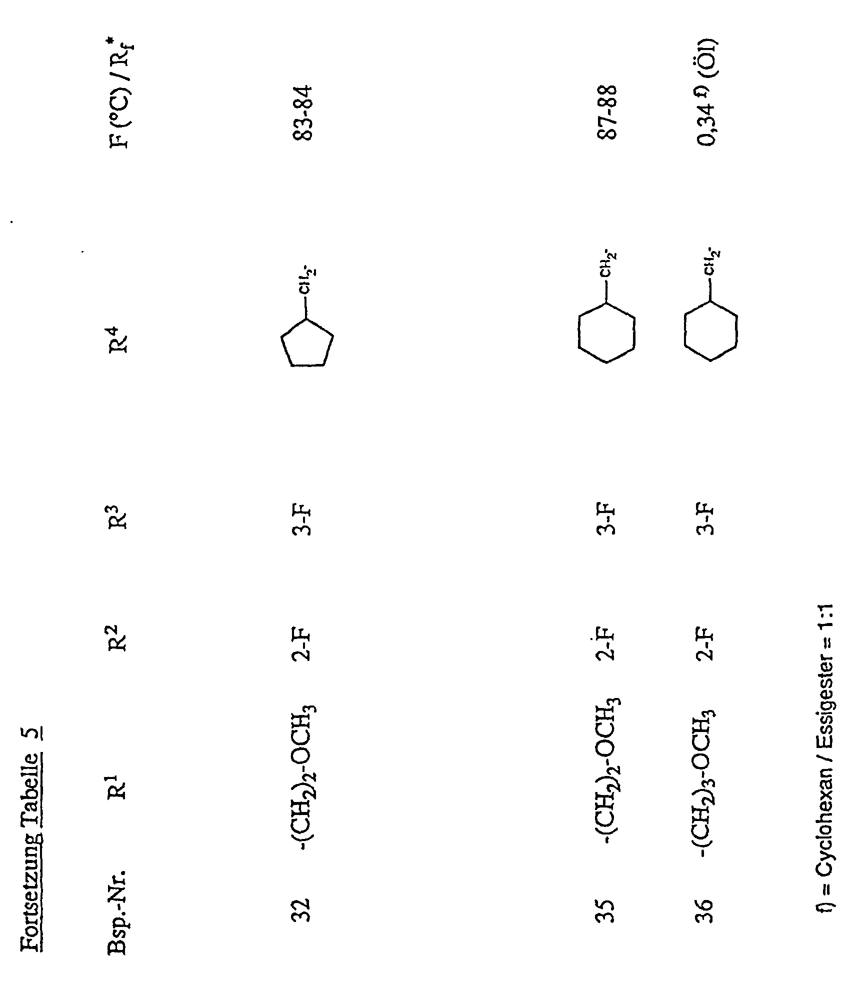

## Patentansprüche

1. 4-Phenyl-3-substituierte 1,4-Dihydropyridinester der allgemeinen Formel 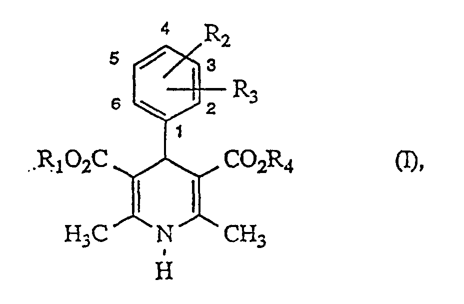 in welcher
R¹ für geradkettiges oder verzweigtes Alkyl mit bis zu 8 Kohlenstoffatomen steht, das gegebenenfalls durch Cycloalkyl mit 3 bis 6 Kohlenstoffatomen oder durch Hydroxy oder durch geradkettiges oder verzweigtes Alkoxy mit bis zu 6 Kohlenstoffatomen substituiert ist, oder
für Cycloalkyl mit 3 bis 8 Kohlenstoffatomen steht,
R² und R³ gleich oder verschieden sind und für Halogen oder Cyano stehen, oder
R² oder R³ für Wasserstoff steht,
R⁴ für geradkettiges oder verzweigtes Alkyl mit bis zu 8 Kohlenstoffatomen steht, das durch Cycloalkyl mit 3 bis 6 Kohlenstoffatomen,
oder durch einen Rest der Formel
-OR⁵ , 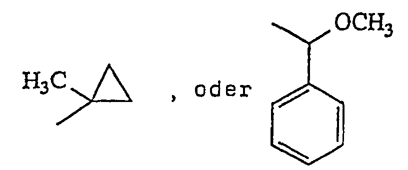
substituiert ist,
worin
R⁵ Cycloalkyl mit 3 bis 6 Kohlenstoffatomen, geradkettiges oder verzweigtes Fluoralkyl mit bis zu 5 Fluoratomen oder geradkettiges oder verzweigtes Alkyl mit bis zu 8 Kohlenstoffatomen bedeutet, das seinerseits durch Methoxy, Ethoxy oder Cycloalkyl mit 3 bis 7 Kohlenstoffatomen substituiert ist,
und deren physiologisch unbedenkliche Salze
mit der Maßgabe, daß R⁴ nicht -(CH₂CH₂O)₂CH₃ bedeutet, wenn
R¹ für -C₂H₅, -CH(CH₃)₂, -C(CH₃)₃, -CH(CH₃)C₂H5, -CH₂CH₂OCH(CH₃)₂, -CH(CH₃)CH₂OCH₃ oder C(CH₃)₂CH₂OCH₃ steht, und
R² und R³ für Chlor stehen.

2. 4-Phenyl-3-substituierte 1,4-Dihydropyridinester nach Anspruch 1,
wobei
R¹ für geradkettiges oder verzweigtes Alkyl mit bis zu 6 Kohlenstoffatomen steht, das gegebenenfalls durch Cyclopropyl, Cyclopentyl, Cyclohexyl, Hydroxy oder durch geradkettiges oder verzweigtes Alkoxy mit bis zu 5 Kohlenstoffatomen substituiert ist, oder für Cyclopentyl, Cyclohexyl oder Cycloheptyl steht,
R² und R³ gleich oder verschieden sind und für Fluor, Chlor, Brom oder Cyano stehen, oder
R² oder R³ für Wasserstoff steht,
R⁴ für geradkettiges oder verzweigtes Alkyl mit bis zu 6 Kohlenstoffatomen steht, das durch Cyclopropyl, Cyclopentyl, Cyclohexyl oder durch einen Rest der Formel
-OR⁵ , 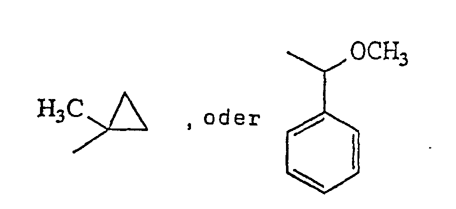
substituiert ist,
worin
R⁵ Cyclopropyl, Cyclopentyl; Cyclohexyl, geradkettiges oder verzweigtes Fluoralkyl mit bis zu 4 Fluoratomen oder geradkettiges oder verzweigtes Alkyl mit bis zu 6 Kohlenstoffatomen bedeutet, das seinerseits durch Methoxy, Ethoxy oder Cyclopropyl substituiert ist
und deren physiologisch unbedenkliche Salze
mit der Maßgabe, daß R⁴ nicht -(CH₂CH₂O)₂CH₃ bedeutet, wenn
R¹ für -C₂H₅, -CH(CH₃)₂, -C(CH₃)₃, -CH(CH₃)C₂H5, -CH₂CH₂OCH(CH₃)₂, -CH(CH₃)CH₂OCH₃ oder C(CH₃)₂CH₂OCH₃ steht, und
R² und R³ für Chlor stehen.

3. 4-Phenyl-3-substituierte 1,4-Dihydropyridinester nach Anspruch 1,
wobei
R¹ für geradkettiges oder verzweigtes Alkyl mit bis zu 5 Kohlenstoffatomen steht, das gegebenenfalls durch Cyclopentyl, Cyclohexyl oder durch Hydroxy oder geradkettiges oder verzweigtes Alkoxy mit bis zu 4 Kohlenstoffatomen substituiert ist, oder
für Cyclopentyl, Cyclohexyl oder Cycloheptyl steht,
R² und R³ gleich oder verschieden sind und für Fluor, Chlor, Brom oder Cyano stehen, oder
entweder R² oder R³ für Wasserstoff steht,
R⁴ für geradkettiges oder verzweigtes Alkyl mit bis zu 5 Kohlenstoffatomen steht, das durch Cyclopropyl, Cyclopentyl, Cyclohexyl, oder durch einen Rest der Formel
-OR⁵ , 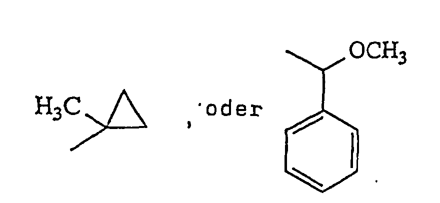
substituiert ist,
worin
R⁵ Cyclopropyl, Cyclopentyl, Cyclohexyl, geradkettiges oder verzweigtes Fluoralkyl mit bis zu 3 Fluoratomen oder geradkettiges oder verzweigtes Alkyl mit bis zu 5 Kohlenstoffatomen bedeutet, das seinerseits durch Methoxy oder Cyclopropyl substituiert ist,
und deren physiologisch unbedenkliche Salze,
mit der Maßgabe, daß R⁴ nicht -(CH₂CH₂O)₂CH₃ bedeutet, wenn
R¹ für -C₂H₅, -CH(CH₃)₂, -C(CH₃)₃, -CH(CH₃)C₂H5, -CH₂CH₂OCH(CH₃)₂ -CH(CH₃)CH₂OCH₃ oder C(CH₃)₂CH₂OCH₃ steht, und
R² und R³ für Chlor stehen.

4. Verbindungen gemäß Formel (I) nach Anspruch 1 aus der Gruppe
Cyclohexylmethyl-2-methoxyethyl-4-(3,4-difluor-phenyl)-1,4-dihydro-2,6-dimethylpyridin-3,5-dicarboxylat,
2-Cyclohexylethyl-2-methoxyethyl-4-(3,4-difluor-phenyl)-1,4-dihydro-2,6-dimethylpyridin-3,5-dicarboxylat,
3-Cyclohexylpropyl-2-methoxyethyl-4-(3,4-difluor-phenyl)-1,4-dihydro-2,6-dimethylpyridin-3,5-dicarboxylat,
Isopropyl-2-(2-methoxyethoxy)ethyl-4-(2-chlor-6-fluor-phenyl)-1,4-dihydro-2,6-dimethylpryridin-3,5-dicarboxylat,
Cyclopentyl-2-(2-methoxyethoxy)ethyl-4-(2-chlor-6-fluor-phenyl)-1,4-dihydro-2,6-dimethylpyridin-3,5-dicarboxylat,
2-Cyclohexyloxyethyl-isopropyl-4-(3-cyano-phenyl)-1,4-dihydro-2,6-dimethylpyridin-3,5-dicarboxylat,
2-Cyclohexyloxyethyl-cyclopentyl-4-(3-cyano-phenyl)-1,4-dihydro-2,6-dimethylpyridin-3,5-dicarboxylat,
Cyclohexylmethyl-2-methoxyethyl-4-(3-cyano-phenyl)-1,4-dihydro-2,6-dimethylpyridin-3,5-dicarboxylat,
Cyclopropylmethyl-2-methoxyethyl-4-(3-chlor-2-cyano-phenyl)-1,4-dihydro-2,6-dimethylpyridin-3,5-dicarboxylat,
2-Methoxyethyl-(1-methyl-cyclopropyl)methyl-4-(3-chlor-2-cyano-phenyl)-1,4-dihydro-2,6-dimethylpyridin-3,5-dicarboxylat,
Isopropyl-(S)-methoxy-2-phenylethyl-4-(2,3-difluor-phenyl)-1,4-dihydro-2,6-dimethylpyridin-3,5-dicarboxylat,
Cyclopropyl-(S)-2-methoxy-2-phenylethyl-4-(2,3-difluor-phenyl)-1,4-dihydro-2,6-dimethylpyridin-3,5-dicarboxylat,
Isopropyl-(R)-2-methoxy-2-phenylethyl-4-(2,3-difluor-phenyl)-1,4-dihydro-2,6-dimethylpyridin-3,5-dicarboxylat,
Cyclopentylmethyl-2-methoxyethyl-4-(2,3-difluor-phenyl)-1,4-dihydro-2,6-dimethylpyridin-3,5-dicarboxylat,
Cyclohexylmethyl-2-methoxyethyl-4-(2,3-difluor-phenyl)-1,4-dihydro-2,6-dimethylpyridin-3,5-dicarboxylat,
Cyclohexylmethyl-2-methoxypropyl-4-(2,3-difluor-phenyl)-1,4-dihydro-2,6-dimethylpyridin-3,5-dicarboxylat,

5. 1,4-Dihydropyridinester nach Ansprüchen 1 bis 4zur Verwendung bei der Bekämpfung von Krankheiten.

6. Verfahren zur Herstellung von 4-Phenyl-3-substituierten 1,4-Dihydropyridinestem nach Ansprüchen 1 bis 4,**dadurch gekennzeichnet, daß** man
[A] Aldehyde der allgemeinen Formel (II) 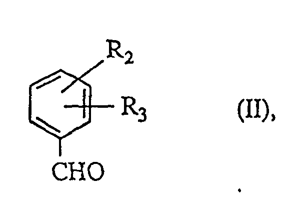 in welcher
R² und R³ die angegebene Bedeutung haben,
zunächst mit Acetessigestem der allgemeinen Formel (III)
H₃C-CO-CH₂-CO₂R¹ (III),
in welcher
R¹ die angegebene Bedeutung hat,
gegebenenfalls unter Isolierung der entsprechenden Ylidenverbindungen der allgemeinen Formel (IV) 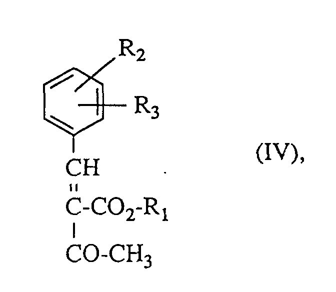 in welcher
R¹, R² und R³ die angegebene Bedeutung haben,
umsetzt und anschließend entweder mit Verbindungen der allgemeinen Formel (V)
CH₃-CO-CH₂-CO₂R⁴ (V),
in welcher
R⁴ die angegebene Bedeutung hat,
in inerten Lösemitteln, in Anwesenheit von Ammoniak oder Ammoniumsalzen,
oder direkt mit Enaminoderivaten der allgemeinen Formel (VI) 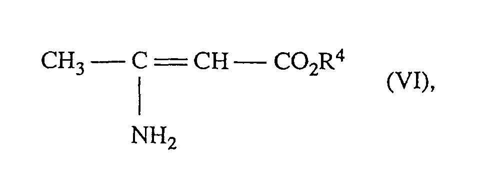 in welcher
R⁴ die angegebene Bedeutung hat,
umsetzt,
oder
[B] die Aldehyde der allgemeinen Formel (II) zunächst mit den Verbindungen der allgemeinen Formel (V), gegebenenfalls unter Isolierung der Ylidenverbindungen der allgemeinen Formel (VII) 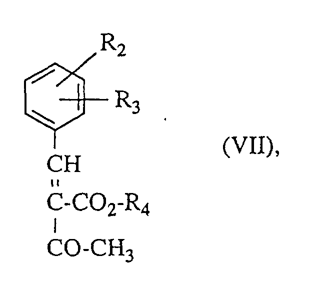 in welcher
R², R³ und R⁴ die angegebene Bedeutung haben,
umsetzt und in einem nächsten Schritt mit den Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII) 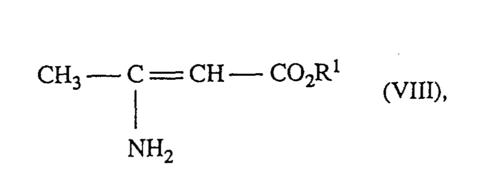 in welcher
R¹ die angegebene Bedeutung hat,
umsetzt,
oder
[C] Verbindungen der allgemeinen Formel (IX) 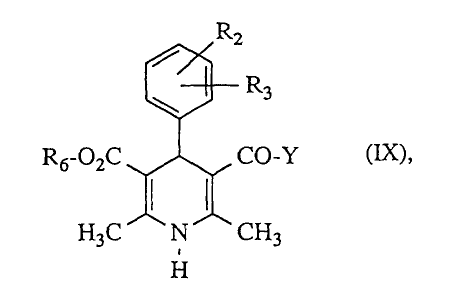 in welcher
R² und R³ die angegebene Bedeutung haben,
R⁶ den angegebenen Bedeutungsumfang von R¹ oder R⁴ umfaßt,
und
Y zusammen mit der -CO-Gruppe eine reaktives Carbonsäurederivat bildet,
in inerten Lösemitteln, in Anwesenheit einer Base,
mit Verbindungen der allgemeinen Formel (X)
R⁷-OH (X),
in welcher
R⁷ die angegebene Bedeutung von R⁶ hat,
umsetzt,
und im Fall der reinen Ester-Enantiomere, die enantiomerenreinen Carbonsäuren, gegebenenfalls zunächst über die Stufe eines reaktiven Säurederivates, mit den entsprechenden Alkoholen umsetzt.

7. Arzneimittel enthaltend mindestens ein 4-Phenyl-3-substituiertes 1,4-Dihydropyridin nach Ansprüchen 1 bis 4.

8. Arzneimittel nach Anspruch 7 zur Behandlung von zentral degenerativen Erkrankungen von Hirn- und Gedächtnisleistungsstörungen, Behandlung oder Prophylaxe von Folgen cerebraler Durchblutungsstörungen sowie zur Behandlung von Depressionen.

9. Verfahren zur Herstellung der Arzneimittel nach Anspruch 8 **dadurch gekennzeichnet, daß** man mindestens einen 4-Phenyl-3-substituierten 1,4-Dihydropyridinester gegebenenfalls unter Verwendung von üblichen Hilfs- und Zusatzstoffen in eine geeignete Applikationsform überführt.

10. Verwendung von 4-Phenyl-3-substituierten 1,4-Dihydropyridinester nach Anspruch 1 zur Herstellung von Arzneimitteln zur Behandlung von zentral degenerativen Erkrankungen von Hirnleistungs- und Gedächtnisstörungen, cerebralen Durchblutungsstörungen und Depressionen.

11. Verwendung von 4-Phenyl-3-substituierte 1,4-Dihydropyridinestem der allgemeinen Formel 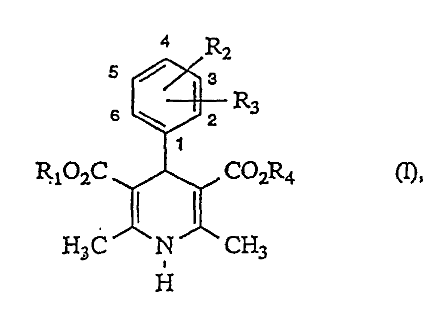 in welcher
R¹ für geradkettiges oder verzweigtes Alkyl mit bis zu 8 Kohlenstoffatomen steht, das gegebenenfalls durch Cycloalkyl mit 3 bis 6 Kohlenstoffatomen oder durch Hydroxy oder durch geradkettiges oder verzweigtes Alkoxy mit bis zu 6 Kohlenstoffatomen substituiert ist, oder
für Cycloalkyl mit 3 bis 8 Kohlenstoffatomen steht,
R² und R³ gleich oder verschieden sind und für Halogen oder Cyano stehen, oder
R² oder R³ für Wasserstoff steht,
R⁴ für geradkettiges oder verzweigtes Alkyl mit bis zu 8 Kohlenstoffatomen steht, das durch Cycloalkyl mit 3 bis 6 Kohlenstoffatomen,
oder durch einen Rest der Formel
-OR⁵ , 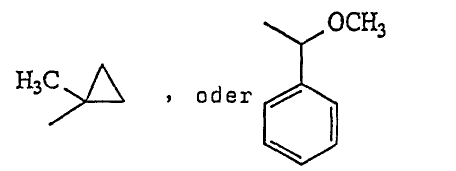
substituiert ist,
worin
R⁵ Cycloalkyl mit 3 bis 6 Kohlenstoffatomen, geradkettiges oder verzweigtes Fluoralkyl mit bis zu 5 Fluoratomen oder geradkettiges oder verzweigtes Alkyl mit bis zu 8 Kohlenstoffatomen bedeutet, das seinerseits durch Methoxy, Ethoxy oder Cycloalkyl mit 3 bis 7 Kohlenstoffatomen substituiert ist,
zur Herstellung von Arzneimitteln zur Behandlung von zentral degenerativen Erkrankungen von Hirnleistungs- und Gedächtnisstörungen, cerebralen Durchblutungsstörungen und Depressionen.

## Claims

1. 4-Phenyl-3-substituted 1,4-dihydropyridine esters of the general formula 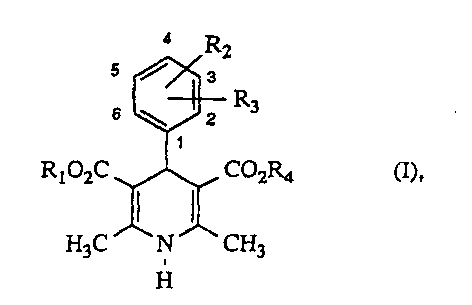 in which
R¹ represents straight-chain or branched alkyl having up to 8 carbon atoms, which is optionally substituted by cycloalkyl having 3 to 6 carbon atoms or by hydroxyl or by straight-chain or branched alkoxy having up to 6 carbon atoms, or
represents cycloalkyl having 3 to 8 carbon atoms,
R² and R³ are identical or different and represent halogen or cyano, or
R² or R³ represents hydrogen,
R⁴ represents straight-chain or branched alkyl having up to 8 carbon atoms, which is substituted by cycloalkyl having 3 to 6 carbon atoms or by a radical of the formula
-OR⁵ , 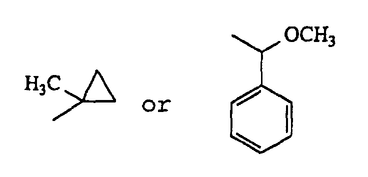
wherein
R⁵ denotes cycloalkyl having 3 to 6 carbon atoms, straight-chain or branched fluoroalkyl having up to 5 fluorine atoms or straight-chain or branched alkyl having up to 8 carbon atoms, which in turn is substituted by methoxy, ethoxy or cycloalkyl having 3 to 7 carbon atoms,
and physiologically acceptable salts thereof,
with the proviso that R⁴ is not
- (CH₂CH₂O)₂CH₃ if R¹ represents
-C₂H₅, -CH(CH₃)₂, -C(CH₃)₃,
-CH(CH₃)C₂H₅, -CH₂CH₂OCH(CH₃)₂,
-CH(CH₃)CH₂OCH₃ or C (CH₃)₂CH₂OCH₃,
and
R² and R³ represent chlorine.

2. 4-Phenyl-3-substituted 1,4-dihydropyridine esters according to Claim 1,
wherein
R¹ represents straight-chain or branched alkyl having up to 6 carbon atoms, which is optionally substituted by cyclopropyl, cyclopentyl, cyclohexyl, hydroxyl or by straight-chain or branched alkoxy having up to 5 carbon atoms, or
represents cyclopentyl, cyclohexyl or cycloheptyl,
R² and R³ are identical or different and represent fluorine, chlorine, bromine or cyano, or
R² or R³ represents hydrogen,
R⁴ represents straight-chain or branched alkyl having up to 6 carbon atoms, which is substituted by cyclopropyl, cyclopentyl, cyclohexyl or by a radical of the formula
-OR⁵, 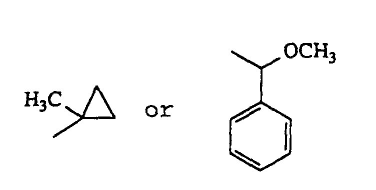
wherein
R⁵ denotes cyclopropyl, cyclopentyl, cyclohexyl, straight-chain or branched fluoroalkyl having up to 4 fluorine atoms or straight-chain or branched alkyl having up to 6 carbon atoms, which in turn is substituted by methoxy, ethoxy or cyclopropyl,
and physiologically acceptable salts thereof,
with the proviso that R⁴ is not
-(CH₂CH₂O)₂CH₃ if R¹ represents
-C₂H₅, -CH(CH₃)₂, -C(CH₃)₃,
-CH(CH₃)C₂H₅, -CH₂CH₂OCH(CH₃)₂,
-CH(CH₃)CH₂OCH₃ or C(CH₃)₂CH₂OCH₃,
and
R² and R³ represent chlorine.

3. 4-Phenyl-3-substituted 1,4-dihydropyridine esters according to Claim 1,
wherein
R¹ represents straight-chain or branched alkyl having up to 5 carbon atoms, which is optionally substituted by cyclopentyl, cyclohexyl or by hydroxyl or straight-chain or branched alkoxy having up to 4 carbon atoms, or
represents cyclopentyl, cyclohexyl or cycloheptyl,
R² and R³ are identical or different and represent fluorine, chlorine, bromine or cyano, or
either R² or R³ represents hydrogen,
R⁴ represents straight-chain or branched alkyl having up to 5 carbon atoms, which is substituted by cyclopropyl, cyclopentyl, cyclohexyl or by a radical of the formula
-OR⁵ , 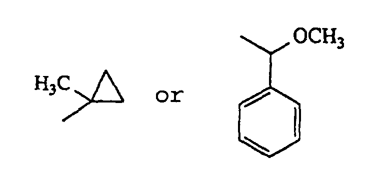
wherein
R⁵ denotes cyclopropyl, cyclopentyl, cyclohexyl, straight-chain or branched fluoroalkyl having up to 3 fluorine atoms or straight-chain or branched alkyl having up to 5 carbon atoms, which in turn is substituted by methoxy or cyclopropyl,
and physiologically acceptable salts thereof,
with the proviso that R⁴ is not
-(CH₂CH₂O)₂CH₃ if R¹ represents
-C₂H₅, -CH(CH₃)₂, -C(CH₃)₃,
-CH(CH₃)C₂H₅, -CH₂CH₂OCH(CH₃)₂,
-CH(CH₃)CH₂OCH₃ or C (CH₃)₂CH₂OCH₃,
and
R² and R³ represent chlorine.

4. Compounds of the formula (I) according to Claim 1, from the group consisting of
cyclohexylmethyl 2-methoxyethyl 4-(3,4-difluorophenyl)-1,4-dihydro-2,6-dimethylpyridine-3,5-dicarboxylate,
2-cyclohexylethyl 2-methoxyethyl 4-(3,4-difluorophenyl)-1,4-dihydro-2,6-dimethylpyridine-3,5-dicarboxylate,
3-cyclohexylpropyl 2-methoxyethyl 4-(3,4-difluorophenyl)-1,4-dihydro-2,6-dimethylpyridine-3,5-dicarboxylate,
isopropyl 2-(2-methoxyethoxy)ethyl 4-(2-chloro-6-fluorophenyl)-1,4-dihydro-2,6-dimethylpyridine-3,5-dicarboxylate,
cyclopentyl 2-(2-methoxyethoxy)ethyl 4-(2-chloro-6-fluorophenyl)-1,4-dihydro-2,6-dimethylpyridine-3,5-dicarboxylate,
2-cyclohexyloxyethyl isopropyl 4-(3-cyanophenyl)-1,4-dihydro-2,6-dimethylpyridine-3,5-dicarboxylate,
2-cyclohexyloxyethyl cyclopentyl 4-(3-cyanophenyl)-1,4-dihydro-2,6-dimethylpyridine-3,5-dicarboxylate,
cyclohexylmethyl 2-methoxyethyl 4-(3-cyanophenyl)-1,4-dihydro-2,6-dimethylpyridine-3,5-dicarboxylate,
cyclopropylmethyl 2-methoxyethyl 4-(3-chloro-2-cyanophenyl)-1,4-dihydro-2,6-dimethylpyridine-3,5-dicarboxylate,
2-methoxyethyl (1-methylcyclopropyl)methyl 4-(3-chloro-2-cyanophenyl)-1,4-dihydro-2,6-dimethylpyridine-3,5-dicarboxylate,
isopropyl (S)-methoxy-2-phenylethyl 4-(2,3-difluorophenyl)-1,4-dihydro-2,6-dimethylpyridine-3,5-dicarboxylate,
cyclopropyl (S)-2-methoxy-2-phenylethyl 4-(2,3-difluorophenyl)-1,4-dihydro-2,6-dimethylpyridine-3,5-dicarboxylate,
isopropyl (R)-2-methoxy-2-phenylethyl 4-(2,3-difluorophenyl)-1,4-dihydro-2,6-dimethylpyridine-3,5-dicarboxylate,
cyclopentylmethyl 2-methoxyethyl 4-(2,3-difluorophenyl)-1,4-dihydro-2,6-dimethylpyridine-3,5-dicarboxylate,
cyclohexylmethyl 2-methoxyethyl 4-(2,3-difluorophenyl)-1,4-dihydro-2,6-dimethylpyridine-3,5-dicarboxylate,
cyclohexylmethyl 2-methoxypropyl 4-(2,3-difluorophenyl)-1,4-dihydro-2,6-dimethylpyridine-3,5-dicarboxylate.

5. 1,4-Dihydropyridine esters according to Claims 1 to 4 for use in combating diseases.

6. Process for the preparation of 4-phenyl-3-substituted 1,4-dihydropyridine esters according to Claims 1 to 4, **characterized in that**
[A] aldehydes of the general formula (II) 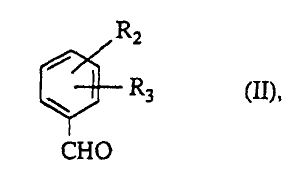 in which
R² and R³ have the meanings given,
are first reacted with acetoacetic acid esters of the general formula (III)
H₃C-CO-CH₂-CO₂R¹ (III)
in which
R¹ has the meaning given,
if appropriate with isolation of the corresponding ylidene compounds of the general formula (IV) 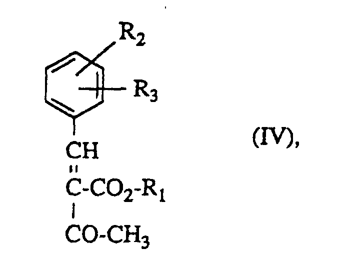 in which
R¹, R² and R³ have the meanings given,
and the products are then either reacted with compounds of the general formula (V)
CH₃-CO-CH₂-CO₂R⁴ (V)
in which
R⁴ has the meaning given,
in inert solvents in the presence of ammonia or ammonium salts,
or are reacted directly with enamino derivatives of the general formula (VI) 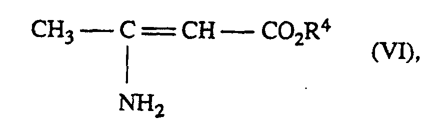 in which
R⁴ has the meaning given,
or
[B] the aldehydes of the general formula (II) are first reacted with the compounds of the general formula (V), if appropriate with isolation of the ylidene compounds of the general formula (VII) 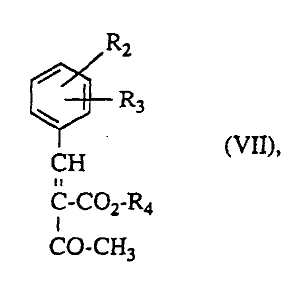 in which
R², R³ and R⁴ have the meanings given,
and in a next step the products are reacted with the compounds of the general formula (III) in inert solvents in the presence of ammonia or ammonium salts, or are reacted directly with enaminocarboxylic acid derivatives of the general formula (VIII) 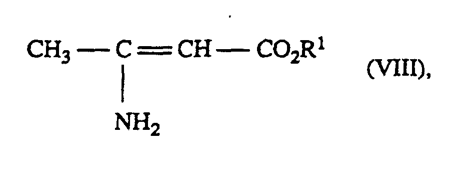 in which
R¹ has the meaning given,
or
[C] compounds of the general formula (IX) 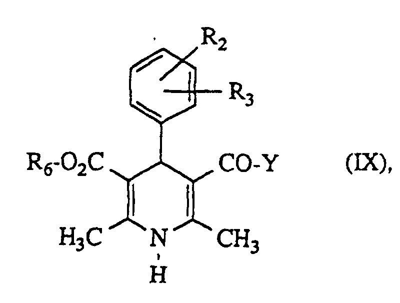 in which
R² and R³ have the meanings given,
R⁶ comprises the scope of meanings given for R¹ or R⁴,
and
Y together with the -CO group forms a reactive carboxylic acid derivative,
are reacted in inert solvents in the presence of a base
with compounds of the general formula (X)
R⁷-OH (X),
in which
R⁷ has the meaning given for R⁶,
and, in the case of the pure ester enantiomers, the enantiomerically pure carboxylic acids are reacted with the corresponding alcohols, if appropriate initially via the stage of a reactive acid derivative.

7. Medicaments comprising at least one 4-phenyl-3-substituted 1,4-dihydropyridine according to Claims 1 to 4.

8. Medicaments according to Claim 7 for the treatment of centrally degenerative diseases of disturbances in cerebral and memory performance, treatment or prophylaxis of the consequences of disturbances in cerebral circulation and for the treatment of depression.

9. Process for the preparation of the medicaments according to Claim 8, **characterized in that** at least one 4-phenyl-3-substituted 1,4-dihydropyridine ester is converted into a suitable administration form, if appropriate using customary auxiliaries and additives.

10. Use of 4-phenyl-3-substituted 1,4-dihydropyridine esters according to Claim 1 for the preparation of medicaments for the treatment of centrally degenerative diseases, of disturbances in cerebral performance and memory, disturbances in cerebral circulation, and depression.

11. Use of 4-phenyl-3-substituted 1,4-dihydropyridine esters of the general formula 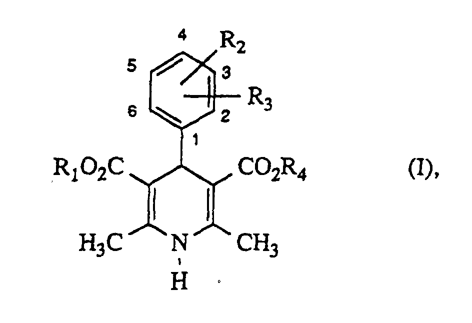 in which
R¹ represents straight-chain or branched alkyl having up to 8 carbon atoms, which is optionally substituted by cycloalkyl having 3 to 6 carbon atoms or by hydroxyl or by straight-chain or branched alkoxy having up to 6 carbon atoms, or
represents cycloalkyl having 3 to 8 carbon atoms,
R² and R³ are identical or different and represent halogen or cyano, or
R² or R³ represents hydrogen,
R⁴ represents straight-chain or branched alkyl having up to 8 carbon atoms, which is substituted by cycloalkyl having 3 to 6 carbon atoms or by a radical of the formula
-OR⁵ , 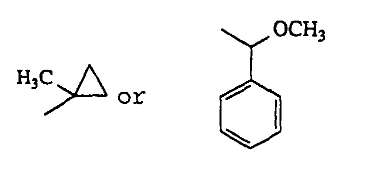
wherein
R⁵ denotes cycloalkyl having 3 to 6 carbon atoms, straight-chain or branched fluoroalkyl having up to 5 fluorine atoms or straight-chain or branched alkyl having up to 8 carbon atoms, which in turn is substituted by methoxy, ethoxy or cycloalkyl having 3 to 7 carbon atoms,
for the preparation of medicaments for the treatment of centrally degenerative diseases, of disturbances in cerebral and memory performance, disturbances in cerebral circulation and depression.

## Revendications

1. Esters de 4-phényl-1,4-dihydropyridine 3-substitués de la formule générale : 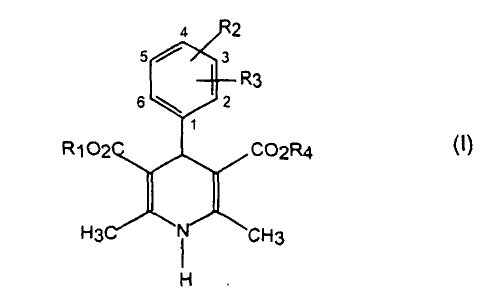 dans laquelle :
R¹ représente un radical alcoyle linéaire ou ramifié ayant jusqu'à 8 atomes de carbone, qui est le cas échéant, substitué par un radical cycloalcoyle ayant 3 à 6 atomes de carbone ou par le radical hydroxyle ou par un radical alcoxy linéaire ou ramifié ayant jusqu'à 6 atomes de carbone, ou représente un radical cycloalcoyle ayant 3 à 8 atomes de carbone ;
R² et R³ sont identiques ou différents et représentent un atome d'halogène ou le radical cyano, ou
R² ou R³ représentent l'atome d'hydrogène ;
R⁴ représente un radical alcoyle linéaire ou ramifié ayant jusqu'à 8 atomes de carbone, qui est substitué par un radical cycloalcoyle ayant 3 à 6 atomes de carbone ou par un reste de la formule :
-OR⁵ , 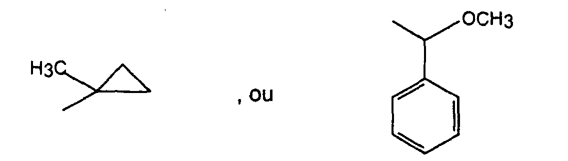
où
R⁵ représente un radical cycloalcoyle ayant 3 à 6 atomes de carbone, un radical fluoroalcoyle linéaire ou ramifié ayant jusqu'à 5 atomes de fluor ou un radical alcoyle linéaire ou ramifié ayant jusqu'à 8 atomes de carbone, qui est substitué à son tour par le radical méthoxy, éthoxy ou un radical cycloalcoyle ayant 3 à 7 atomes de carbone,
et leurs sels physiologiquement sans inconvénient,
avec la condition que R⁴ ne signifie pas -(CH₂CH₂O)₂CH₃, lorsque R¹ représente -C₂H₅, -CH(CH₃)₂, -C(CH₃)₃, -CH(CH₃)C₂H₅, -CH₂CH₂OCH(CH₃)₂, -CH (CH₃)CH₂OCH₃ ou -C(CH₃)₂CH₂OCH₃, et
R² et R³ représentent l'atome de chlore.

2. Esters de 4-phényl-1,4-dihydropyridine 3-substitués suivant la revendication 1, où
R¹ représente un radical alcoyle linéaire ou ramifié ayant jusqu'à 6 atomes de carbone, qui est le cas échéant, substitué par le radical cyclopropyle, cyclopentyle, cyclohexyle, par le radical hydroxyle ou par un radical alcoxy linéaire ou ramifié ayant jusqu'à 5 atomes de carbone, ou représente le radical cyclopentyle, cyclohexyle ou cycloheptyle ;
R² et R³ sont identiques ou différents et représentent l'atome de fluor, de chlore, de brome ou le radical cyano, ou
R² ou R³ représentent l'atome d'hydrogène ;
R⁴ représente un radical alcoyle linéaire ou ramifié ayant jusqu'à 6 atomes de carbone, qui est substitué par le radical cyclopropyle, cyclopentyle, cyclohexyle, ou par un reste de la formule :
-OR⁵, 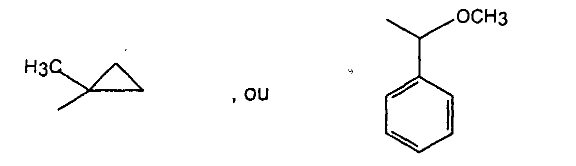
où
R⁵ représente le radical cyclopropyle, cyclopentyle, cyclohexyle, un radical fluoroalcoyle linéaire ou ramifié ayant jusqu'à 4 atomes de fluor ou un radical alcoyle linéaire ou ramifié ayant jusqu'à 6 atomes de carbone, qui est substitué à son tour par le radical méthoxy, éthoxy ou le radical cyclopropyle,
et leurs sels physiologiquement sans inconvénient,
avec la condition que R⁴ ne signifie pas -(CH₂CH₂O)₂CH₃, lorsque R¹ représente -C₂H₅, -CH(CH₃)₂, -C(CH₃)₃, -CH(CH₃)C₂H₅, -CH₂CH₂OCH(CH₃)₂, -CH(CH₃)CH₂OCH₃ ou -C(CH₃)₂CH₂OCH₃, et
R² et R³ représentent l'atome de chlore.

3. Esters de 4-phényl-1,4-dihydropyridine 3-substitués suivant la revendication 1, où
R¹ représente un radical alcoyle linéaire ou ramifié ayant jusqu'à 5 atomes de carbone, qui est le cas échéant, substitué par le radical cyclopentyle, cyclohexyle ou par le radical hydroxyle ou par un radical alcoxy linéaire ou ramifié ayant jusqu'à 4 atomes de carbone, ou représente le radical cyclopentyle, cyclohexyle ou cycloheptyle ;
R² et R³ sont identiques ou différents et représentent l'atome de fluor, de chlore, de brome ou le radical cyano, ou
R² ou R³ représentent l'atome d'hydrogène ;
R⁴ représente un radical alcoyle linéaire ou ramifié ayant jusqu'à 5 atomes de carbone, qui est substitué par le radical cyclopropyle, cyclopentyle, cyclohexyle, ou par un reste de la formule :
-OR⁵, 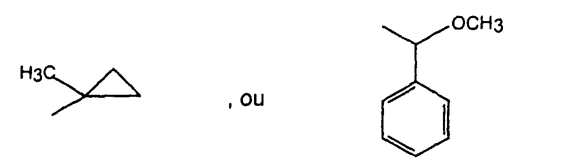
où
R⁵ représente le radical cyclopropyle, cyclopentyle, cyclohexyle, un radical fluoroalcoyle linéaire ou ramifié ayant jusqu'à 3 atomes de fluor ou un radical alcoyle linéaire ou ramifié ayant jusqu'à 5 atomes de carbone, qui est substitué à son tour par le radical méthoxy ou cyclopropyle,
et leurs sels physiologiquement sans inconvénient,
avec la condition que R⁴ ne signifie pas -(CH₂CH₂O)₂CH₃, lorsque R¹ représente -C₂H₅, -CH(CH₃)₂, -C(CH₃)₃, -CH(CH₃)C₂H₅, -CH₂CH₂OCH(CH₃)₂, -CH(CH₃)CH₂OCH₃ ou -C(CH₃)₂CH₂OCH₃, et
R² et R³ représentent l'atome de chlore.

4. Composés selon la formule (I) suivant la revendication 1, parmi le groupe composé de :
le 4-(3,4-difluorophényl)-1,4-dihydro-2,6-diméthylpyridine-3,5-dicarboxylate de cyclohexylméthyle et de 2-méthoxyéthyle,
le 4-(3,4-difluorophényl)-1,4-dihydro-2,6-diméthylpyridine-3,5-dicarboxylate de 2-cyclohexyléthyle et de 2-méthoxyéthyle,
le 4-(3,4-difluorophényl)-1,4-dihydro-2,6-diméthylpyridine-3,5-dicarboxylate de 3-cyclohexylpropyle et de 2-méthoxyéthyle,
le 4-(2-chloro-6-fluorophényl)-1,4-dihydro-2,6-diméthylpyridine-3,5-dicarboxylate d'isopropyle et de 2-(2-méthoxyéthoxy)éthyle,
le 4-(2-chloro-6-fluorophényl)-1,4-dihydro-2,6-diméthylpyridine-3,5-dicarboxylate de cyclopentyle et de 2-(2-méthoxyéthoxy)éthyle,
le 4-(3-cyanophényl)-1,4-dihydro-2,6-diméthylpyridine-3,5-dicarboxylate de 2-cyclohexyloxyéthyle et d'isopropyle,
le 4-(3-cyanophényl)-1,4-dihydro-2,6-diméthylpyridine-3,5-dicarboxylate de 2-cyclohexyloxyéthyle et de cyclopentyle,
le 4- (3-cyanophényl)-1,4-dihydro-2,6-diméthylpyridine-3,5-dicarboxylate de cyclohexylméthyle et de 2-méthoxyéthyle,
le 4-(3-chloro-2-cyanophényl)-1,4-dihydro-2,6-diméthylpyridine-3,5-dicarboxylate de cyclopropylméthyle et de 2-méthoxyéthyle,
le 4-(3-chloro-2-cyanophényl)-1,4-dihydro-2,6-diméthylpyridine-3,5-dicarboxylate de 2-méthoxyéthyle et de (1-méthylcyclopropyl)méthyle,
le 4-(2,3-difluorophényl)-1,4-dihydro-2,6-diméthylpyridine-3,5-dicarboxylate d'isopropyle et de (S)-méthoxy-2-phényléthyle,
le 4-(2,3-difluorophényl)-1,4-dihydro-2,6-diméthylpyridine-3,5-dicarboxylate de cyclopropyle et de (S)-2-méthoxy-2-phényléthyle,
le 4-(2,3-difluorophényl)-1,4-dihydro-2,6-diméthylpyridine-3, 5-dicarboxylate d'isopropyle et de (R)-2-méthoxy-2-phényléthyle,
le 4-(2,3-difluorophényl)-1,4-dihydro-2,6-diméthylpyridine-3,5-dicarboxylate de cyclopentylméthyle et de 2-méthoxyéthyle,
le 4-(2,3-difluorophényl)-1,4-dihydro-2,6-diméthylpyridine-3,5-dicarboxylate de cyclohexylméthyle et de 2-méthoxyéthyle,
le 4-(2,3-difluorophényl)-1,4-dihydro-2,6-diméthylpyridine-3,5-dicarboxylate de cyclohexylméthyle et de 2-méthoxypropyle.

5. Ester de 1,4-dihydropyridine suivant les revendications 1 à 4, à utiliser pour lutter contre des maladies.

6. Procédé de préparation d'esters de 4-phényl-1,4-dihydropyridine 3-substitués suivant les revendications 1 à 4, **caractérisé en ce que** :
[A] on fait réagir des aldéhydes de la formule générale (II) : 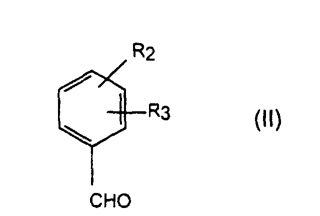 dans laquelle
R² et R³ ont la signification indiquée,
d'abord avec des esters de l'acide acétoacétique de la formule générale (III) :
H₃C-CO-CH₂-CO₂R¹ (III)
dans laquelle
R¹ a la signification indiquée,
le cas échéant, avec isolement des composés ylure correspondants de la formule générale (IV) : 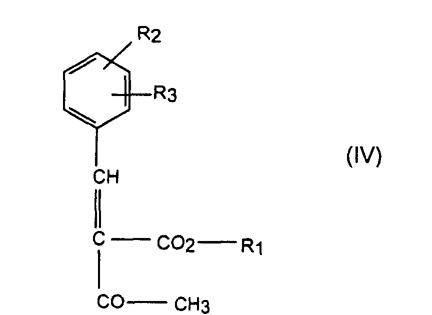 dans laquelle :
R¹, R² et R³ ont la signification indiquée,
et ensuite, on fait réagir avec des composés de la formule générale (V) :
CH₃ - CO - CH₂ - CO₂R⁴ (V)
dans laquelle
R⁴ a la signification indiquée,
dans un solvant inerte, en présence d'ammoniac ou de sels d'ammonium,
ou directement avec des dérivés d'énamine de la formule générale (VI) : 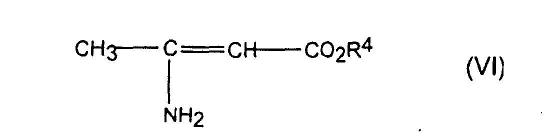 dans laquelle
R⁴ a la signification indiquée,
ou
[B] on fait réagir des aldéhydes de la formule générale (II), d'abord avec les composés de la formule générale (V), le cas échéant en isolant les composés ylure de la formule générale (VII) : 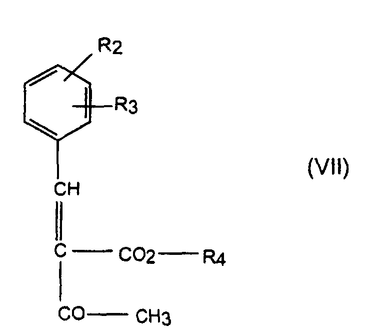 dans laquelle :
R¹, R² et R³ ont la signification indiquée,
et dans une étape suivante, on fait réagir avec les composés de la formule générale (III) dans un solvant inerte, en présence d'ammoniac ou de sels d'ammonium ou directement avec les dérivés acide énaminecarboxylique de la formule générale (VIII) : 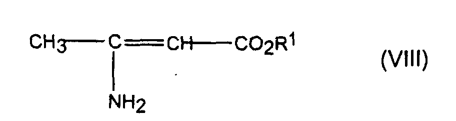
dans laquelle
R¹ a la signification indiquée,
ou
[C] on fait réagir des composés de la formule générale (IX) : 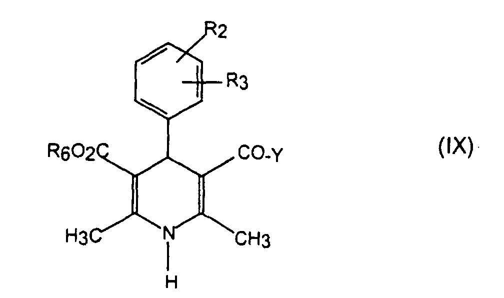
dans laquelle
R² et R³ ont la signification indiquée,
R⁶ comprend les domaines de signification de R¹ ou de R⁴, et
Y forme avec le radical CO, un dérivé réactif d'acide carboxylique,
dans un solvant inerte, en présence d'une base, avec des composés de la formule générale (X) :
R⁷ - OH (X)
dans laquelle
R⁷ a la signification indiquée pour R⁶,
et dans le cas de l'énantiomère pur de l'ester, on fait réagir les acides carboxyliques énantiomériquement purs, le cas échéant d'abord par l'étape d'un dérivé réactif d'acide, avec les alcools appropriés.

7. Agent pharmaceutique contenant au moins une 4-phényl-1,4-dihydropyridine 3-substituée suivant les revendications 1 à 4.

8. Agent pharmaceutique suivant la revendication 7, pour le traitement de maladies dégénératives centrales de troubles de capacité cérébrale et de la mémoire, le traitement ou la prophylaxie des suites de troubles vasculaires cérébraux ainsi que pour le traitement des dépressions.

9. Procédé de préparation de l'agent pharmaceutique suivant la revendication 8, **caractérisé en ce que** l'on convertit au moins un ester de 4-phényl-1,4-dihydropyridine 3-substitué, le cas échéant en utilisant les auxiliaires et additifs usuels, en une forme appropriée d'administration.

10. Utilisation d'esters de 4-phényl-1,4-dihydropyridine 3-substitués suivant la revendication 1, pour la préparation d'agents pharmaceutiques pour le traitement de maladies dégénératives centrales de troubles de capacité cérébrale et de la mémoire, des troubles vasculaires cérébraux et des dépressions.

11. Utilisation d'esters de 4-phényl-1,4-dihydropyridine 3-substitués de la formule générale : 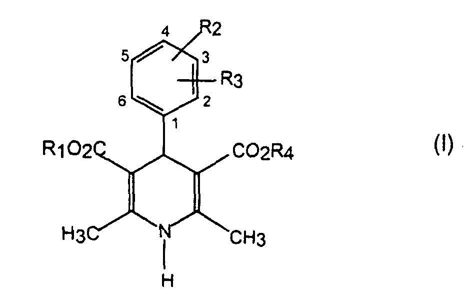 dans laquelle :
R¹ représente un radical alcoyle linéaire ou ramifié ayant jusqu'à 8 atomes de carbone, qui est le cas échéant, substitué par un radical cycloalcoyle ayant 3 à 6 atomes de carbone ou par le radical hydroxyle ou par un radical alcoxy linéaire ou ramifié ayant jusqu'à 6 atomes de carbone, ou représente un radical cycloalcoyle ayant 3 à 8 atomes de carbone ;
R² et R³ sont identiques ou différents et représentent un atome d'halogène ou le radical cyano, ou
R² ou R³ représentent l'atome d'hydrogène ;
R⁴ représente un radical alcoyle linéaire ou ramifié ayant jusqu'à 8 atomes de carbone, qui est substitué par un radical cycloalcoyle ayant 3 à 6 atomes de carbone ou par un reste de la formule :
-OR⁵, 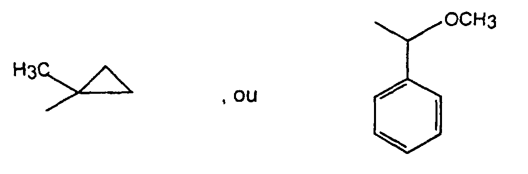
où
R⁵ représente un radical cycloalcoyle ayant 3 à 6 atomes de carbone, un radical fluoroalcoyle linéaire ou ramifié ayant jusqu'à 5 atomes de fluor ou un radical alcoyle linéaire ou ramifié ayant jusqu'à 8 atomes de carbone, qui est substitué à son tour, par le radical méthoxy, éthoxy ou un radical cycloalcoyle ayant 3 à 7 atomes de carbone,
pour la préparation d'agents pharmaceutiques pour le traitement de maladies dégénératives centrales de troubles de capacité cérébrale et de la mémoire, des troubles vasculaires cérébraux et des dépressions.
